# FASCICULE DE BREVET EUROPEEN

(11) **EP 1 542 988 B1**
(45) Date de publication et mention de la délivrance du brevet: **12.04.2006**
(21) Numéro de dépôt: 03769605.1
(22) Date de dépôt: 10.09.2003
(51) Int. Cl.: C07D 401/06, A61K 31/4709, A61P 31/04, C07D 409/14, C07D 211/34

(54) **DERIVES DE LA QUINOLYL PROPYL PIPERIDINE ET LEUR UTILISATION EN TANT QU'ANTIMICROBIENS**
CHINOLYL-PROPYLPIPERIDINDERIVATE UND DEREN VERWENDUNG ALS ANTIMIKROBIELLES MITTEL
PIPERIDINE QUINOLYL PROPYL DERIVATIVES, PREPARATION METHOD AND INTERMEDIATES AND COMPOSITIONS CONTAINING SAME

(30) Priorité: 11.09.2002 FR 0211212
(43) Date de publication de la demande: 22.06.2005
(73) Titulaire: NOVEXEL, 93230 Romainville (FR)
(72) Inventeur: BACQUE, Eric, F-91190 Gif sur Yvette (FR); BIGOT, Antony, F-91300 Massy (FR); EL AHMAD, Youssef, F-94000 Creteil (FR); RONAN, Baptiste, F-92140 Clamart (FR); TABART, Michel, F-91290 La Norville (FR); VIVIANI, Fabrice, F-95380 Louvres (FR); MALLERON, Jean-Luc, F-91460 Marcoussis (FR); MIGNANI, Serge, F-92290 Châtenay-Malabry (FR)
(74) Mandataire: Hirsch & Associés
(86) Numéro de dépôt international: PCT/FR2003/002686
(87) Numéro de publication internationale: WO 2004/024712

(56) Documents cités:
- WO-A-00/43383
- WO-A-01/25227
- WO-A-02/40474

## Description

La présente invention concerne des dérivés de quinolyl propyl pipéridine de formule générale (I) : 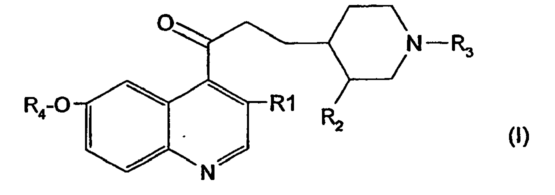 qui sont actifs comme antimicrobiens. L'invention concerne également procédé et intermédiaires de préparation et les compositions les contenant.

Dans les demandes de brevet WO 99/37635 et WO 00/43383 ont été décrits des dérivés de quinolyl propyl pipéridine antimicrobiens, de formule générale : 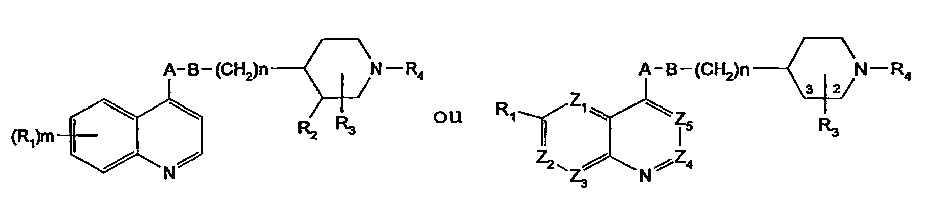 dans laquelle le radical R₁ est notamment alcoxy (C1-6), R₂ est hydrogène, R₃ est en position -2 ou -3 et représente alcoyle (C1-6) pouvant être éventuellement substitué par 1 à 3 substituants choisis parmi thiol, halogène, alcoylthio, trifluorométhyl, carboxy, alcoyloxycarbonyle, alcoylcarbonyle, alcènyloxycarbonyle, alcènylcarbonyle, hydroxy éventuellement substitué par alcoyle ..., R₄ est un groupe -CH₂-R₅ pour lequel R₅ est selectionné parmi alcoyle hydroxyalcoyle, alcènyle, alcynyle, tétrahydrofuryle, phénylalcoyle éventuellement substitué, phénylalcényle éventuellement substitué, hétéroarylalcoyle éventuellement substitué, hétéroaroyle éventuellement substitué ..., n est 0 à 2, m est 1 ou 2 et A et B sont notamment oxygène, soufre, sulfinyle, sulfonyle, NR₁₁, CR₆R₇ pour lequel R₆ et R₇ représentent H, thiol, alcoylthio, halo, trifluorométhyle, alcènyle, alcènylcarbonyle, hydroxy, amino, et Z₁ à Z₅ sont N ou CR₁ₐ ...

Par ailleurs, les demandes de brevets WO-A-01 25227 et WO-A-02 40474 décrivent respectivement des dérivés de type quinolyl propyl pipéridine 3-carboxy ou 3-hydroxy méthyle diversement substitués sur la quinoléine mais ne comportant pas de groupe oxo en position 1 du radical propyle fixé en position 4 de la quinoléine et des dérivés de type quinolyl propyl pipéridine 4-carboxy ou 4-hydroxyméthyle également diversement substitués sur la quinoléine, ne comportant pas non plus de groupe oxo en position 1 du radical propyle.

Enfin, dans la demande de brevet européen EP30044 ont été décrits des dérivés de quinoléine utiles comme cardiovasculaires, répondant à la formule générale : 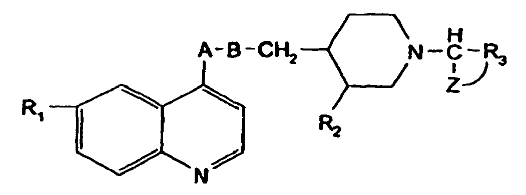 dans laquelle R₁ est notamment alcoyloxy, A-B est -CH₂-CH₂-, -CHOH-CH₂-, -CH₂-CHOH-, -CH₂-CO- ou -CO-CH₂-, R₁ est H, OH ou alcoyloxy, R₂ est éthyle ou vinyle, R₃ est notamment alcoyle, hydroxyalcoyle, cycloalcoyle, hydroxy, alcènyle, alcynyle, tétrahydrofuryle, phénylalcoyle, diphénylalcoyle éventuellement substitué, phénylalcényle éventuellement substitué, benzoyl ou benzoylalcoyle éventuellement substitué, hétéroaryle ou hétéroarylalcoyle éventuellement substitué et Z est H ou alcoyle ou forme avec R₃ un radical cycloalcoyle.

Il a maintenant été trouvé, et c'est ce qui fait l'objet de la présente invention, que les produits de formule générale (I) pour lesquels :
R₁ est un atome d'hydrogène ou de fluor,
R₂ représente un radical carboxy, carboxyméthyle ou hydroxyméthyle,
R₃ représente un radical alcoyle (1 à 6 atomes de carbone) substitué par un radical phénylthio pouvant lui même porter 1 à 4 substituants choisis dans le groupe constitué par halogène, hydroxy, alcoyle, alcoyloxy, trifluorométhyle, trifluorométhoxy, carboxy, alcoyloxycarbonyle, cyano et amino, par un radical cycloalcoylthio dont la partie cyclique contient 3 à 7 chaînons, pouvant lui-même porter un ou plusieurs substituants choisis parmi halogène et trifluorométhyle, ou par un radical hétéroarylthio de 5 à 6 chaînons comprenant 1 à 4 hétéroatomes choisis parmi l'azote, l'oxygène et le soufre, pouvant lui-même porter un ou plusieurs substituants choisis dans le groupe constitué par halogène, hydroxy, alcoyle, alcoyloxy, trifluorométhyle, trifluorométhoxy, carboxy, alcoyloxycarbonyle, cyano et amino ou R₃ représente un radical propargyle substitué par un radical phényle pouvant lui même porter 1 à 4 substituants choisis dans le groupe constitué par halogène, hydroxy, alcoyle, alcoyloxy, trifluorométhyle, trifluorométhoxy, carboxy, alcoyloxycarbonyle, cyano et amino, ou substitué par un radical cycloalcoyle contenant 3 à 7 chaînons pouvant lui-même porter un ou plusieurs substituants choisis parmi halogène et trifluorométhyle, ou substitué par un radical hétéroaryle de 5 à 6 chaînons comprenant 1 à 4 hétéroatomes choisis parmi l'azote, l'oxygène et le soufre et pouvant lui-même porter un ou plusieurs substituants choisis dans le groupe constitué par halogène, hydroxy, alcoyle, alcoyloxy, trifluorométhyle, trifluorométhoxy, carboxy, alcoyloxycarbonyle, cyano et amino, et
R₄ représente un radical alcoyle contenant 1 à 6 atomes de carbone, alcényl-CH₂- ou alcynyl-CH₂- dont les parties alcényle ou alcynyle contiennent 2 à 6 atomes de carbone, cycloalcoyle ou cycloalcoyl alcoyle dont la partie cycloalcoyle contient 3 à 8 atomes de carbone,
sous leurs formes isomères, énantiomères et diastéréoisomères, séparées ou en mélanges, ainsi que leurs sels, sont de puissants agents antibactériens.

Il est entendu que les radicaux et portions alcoyle sont en chaîne droite ou ramifiée et contiennent, sauf mention spéciale, 1 à 4 atomes de carbone, et que lorsque R₃ porte un substituant halogène, celui-ci peut être choisi parmi fluor, chlore, brome et iode, le fluor étant préféré.

Dans la formule générale ci-dessus, lorsque R₃ porte un substituant hétéroaryle, ce dernier peut être choisi, à titre non limitatif, parmi thiényle, furyle, pyrrolyle, imidazolyle, thiazolyle, oxazolyle, thiadiazolyle, oxadiazolyle, tétrazolyle, pyridyle, pyridazinyle, pyrazinyle et pyrimidinyle.

L'invention a notamment pour objet les dérivés de formule générale (I) telle que définie précédemment, dans laquelle R₄ représente un radical alcoyle comportant de 1 à 6 atomes de carbone, notamment méthyle, ceux dans laquelle R₂ représente un radical carboxy et ceux dans laquelle R₃ représente un radical alcoyle, notamment éthyle, substitué par un radical phénylthio, cycloalcoylthio ou hétéroarylthio éventuellement substitués tels que définis plus haut, plus particulièrement ceux dans laquelle R₃ représente un radical éthyle substitué par un radical thiénylthio, phénylthio substitué par halogène, notamment fluor, ou par trifluorométhyle, cyclohexylthio ou cyclopentylthio, et ceux dont les noms suivent :
Acide 1-(2-cyclohexylsulfanyl-éthyl)-4-[3-(3-fluoro-6-méthoxy-quinolin-4-yl)-3-oxo-propyl]-pipéridine-3-carboxylique,
Acide 4-[3-(3-fluoro-6-méthoxy-quinolin-4-yl)-3-oxo-propyl]-1-[3-(2,3,5-trifluoro-phényl)-prop-2-ynyl]-pipéridine-3-carboxylique,
Acide 4-[3-oxo-3-(3-fluoro-6-méthoxyquinolin-4-yl)-propyl]-1-[2-(2,5-difluoro-phénylsulfanyl)-éthyl]-pipéridine-3-carboxylique,
Acide 4-[3-oxo-3-(3-fluoro-6-méthoxyquinolin-4-yl)-propyl]-1-[2-(2,5-difluoro-phénylsulfanyl)-éthyl]-pipéridine-3-acétique,
Acide 4-[3-oxo-3-(6-méthoxyquinolin-4-yl)-propyl]- 1-[2-(2-thiénylsulfanyl)-éthyl]-pipéridine-3-carboxylique,
Acide 4-[3-oxo-3-(6-méthoxyquinolin-4-yl)propyl]-1-[3-(2,3,5-trifluorophényl)prop-2-ynyl]pipéridine-3-carboxylique,
sous leurs différentes formes isomères, séparées ou en mélanges, ainsi que leurs sels.

Selon l'invention, les produits de formule générale (I) peuvent être obtenus selon le procédé A par condensation de la chaîne R₃ sur le dérivé de quinolyl propyl pipéridine de formule générale (II) : 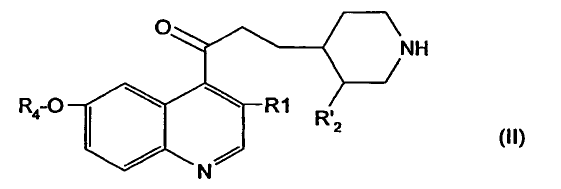 dans laquelle la fonction cétone est, le cas échéant, intermédiairement protégée, R₁ et R₄ sont définis comme précédemment et R'₂ représente un radical carboxy ou carboxyméthyle protégé, pour obtenir un dérivé de quinolyl propyl pipéridine de formule générale (III): 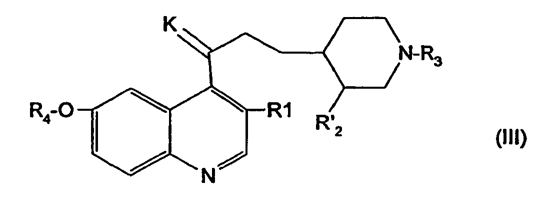 pour lequel R₁, R'₂, R₃ et R₄ sont définis comme ci-dessus et K représent un atome d'oxgyène ou un groupement protecteur de cétone, puis transformation de R'₂ en un radical carboxy ou carboxyméthyl, et/ou, le cas échéant, réduction du radical carboxy ainsi obtenu ou du radical carboxy protégé que peut représenter R'₂ en un radical hydroxyméthyle et éventuellement transformation de celui-ci en un radical carboxyméthyle selon les méthodes habituelles, puis, le cas échéant, séparation des isomères, élimination du radical protecteur d'acide, élimination du radical protecteur de la cétone, et/ou transformation du produit obtenu en un sel.

La condensation de la chaîne R₃ sur la pipéridine s'effectue avantageusement par action d'un dérivé de formule générale :

R₃-X (IV)

dans laquelle R₃ est défini comme précédemment et X représente un atome d'halogène, un radical méthylsulfonyloxy, un radical trifluorométhylsulfonyloxy ou p-toluènesulfonyloxy, en opérant en milieu anhydre, de préférence inerte (azote ou argon par exemple) dans un solvant organique tel qu'un amide (diméthylformamide par exemple), une cétone (acétone par exemple) ou un nitrile (acétonitrile par exemple) en présence d'une base telle qu'une base organique azotée (par exemple triéthylamine) ou une base minérale (carbonate alcalin : carbonate de potassium par exemple) à une température comprise entre 20°C et la température de reflux du solvant. De préférence, on fait agir un dérivé pour lequel X est un atome de brome ou d'iode.

Des dérivés de formule (IV) sont décrits ou peuvent être préparés comme décrit, par exemple, dans les demandes WO20012522 et WO 200240474.

Lorsque R₃ représente propargyle substitué par phényle, cycloalcoyle ou hétéroaryle, il peut être aussi préférable de condenser un halogénure de propargyle, puis de substituer la chaîne par un radical phényle, cycloalcoyle ou hétéroaryle. Dans cette alternative, la condensation de la chaîne propargylique s'effectue au moyen de bromure de propargyle, dans les conditions énoncées ci-dessus, le cas échéant en présence d'un iodure de métal alcalin comme par exemple l'iodure de potassium ou de sodium.
Lorsqu'il s'agit de la substitution par un radical phényle ou hétéroaryle, la réaction s'effectue par action d'un halogénure dérivé du radical cyclique à substituer, en présence de triéthylamine, en milieu anhydre, éventuellement sans solvant ou dans un solvant tel qu'un amide (diméthylformamide par exemple) ou un nitrile (acétonitrile par exemple) et en présence d'un sel de palladium comme par exemple le tétrakis triphénylphosphine palladium et d'iodure cuivreux, à une température comprise entre 20°C et la température de reflux du solvant.
Lorsqu'il s'agit de la substitution par un groupement cycloalkyle, la réaction s'effectue, après protection sous forme d'acétal cyclique ou non de la fonction cétone en position alpha de la quinoléine, par action d'un organolithien comme le n-butyllithium ou le tert-butyllithium sur le dérivé propargylique obtenu précédemment, en milieu anhydre dans un éther comme par exemple le tétrahydrofurane à une température comprise entre -78 et 0°C, puis action d'une cycloalcanone suivi de la désoxygénation de l'alcool intermédiaire et enfin de la déprotection de la fonction cétone selon les méthodes classiques.

Il est entendu que, lorsque les radicaux alcoyle représentés par R₃ portent des substituants carboxy ou amino, ces derniers sont préalablement protégés, puis libérés après la réaction. On opère selon les méthodes habituelles qui n'altèrent pas le reste de la molécule, notamment selon les méthodes décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973).

Le radical carboxy ou carboxyméthyle protégé représenté par R'₂ peut être choisi parmi les esters facilement hydrolysables. A titre d'exemple peuvent être cités les esters méthyliques, benzyliques, tertiobutyliques., ou bien les esters d'allyle ou de phénylpropyle. Le cas échéant, la protection du radical carboxy s'effectue simultanément à la réaction. Dans ce cas le produit de formule générale (II) mis en oeuvre porte un radical R'₂ est un radical carboxy ou carboxyméthyle.

L'élimination, le cas échéant, du radical protecteur d'acide pour obtenir un dérivé de quinolyl propyl pipéridine pour lequel R₂ est un radical carboxy ou carboxyméthyle, s'effectue selon les méthodes habituelles, notamment par hydrolyse acide ou saponification de l'ester R'₂. On fait notamment agir la soude en milieu hydroorganique, par exemple dans un alcool comme le méthanol ou un éther comme le dioxane, à une température comprise entre 20°C et la température de reflux du mélange réactionnel. On peut également mettre en oeuvre une hydrolyse en milieu chlorhydrique aqueux à une température comprise entre 20 et 100°C.

Selon l'invention, le dérivé de formule générale (I) pour lequel R₂ est hydroxyméthyle peut être préparé par action d'un agent de réduction approprié sur un dérivé pour lequel R'₂ est carboxy ou carboxy protégé et la fonction cétone en alpha de la quinoléine est intermédiairement et, également selon l'invention, le dérivé de formule générale (I) pour lequel R2 est carboxyméthyle peut être préparé à partir du dérivé pour lequel R'2 est hydroxyméthyle, obtenu comme décrit ci-dessus, par action sur celui-ci d'un agent d'halogénation ou de tosylation, puis d'un agent de cyanuration et enfin hydrolyse du nitrile.

On peut effectuer la réduction du carboxy selon les méthodes habituelles qui n'altèrent pas le reste de la molécule, en particulier par action d'un hydrure (hydrure d'aluminium et de lithium ou hydrure de diisobutyl aluminium par exemple) dans un solvant tel qu'un éther (tétrahydrofurane par exemple) à une température comprise entre 20 et 60°C. On protège intermédiairement puis déprotège la fonction cétone en position alpha de la quinoléine selon les méthodes classiques connues de l'homme du métier, notamment via un acétal, cyclique ou non.

La réduction de l'acide libre peut être effectuée selon des méthodes également connues de l'homme du métier, par exemple par hydrogénation en présence d'un catalyseur à base de rhodium ou de ruthénium, par action de hydroborure de sodium en présence d'acide de Lewis ou d'hydrure d'aluminium et de lithium dans l'éther. De préférence, la fonction cétone est dans ce cas également intermédiairement protégée.

La transformation du radical hydroxyméthyle en position 3 de la pipéridine en un radical carboxyméthyle s'effectue selon les méthodes habituelles qui n'altèrent pas le reste de la molécule, notamment par action d'un agent d'halogénation comme par exemple le chlorure de thionyle ou le trichlorure de phosphore ou le tribromure de phosphore, ou d'un agent de tosylation, puis d'un cyanure alcalin, par exemple (cyanure de potassium ou cyanure de sodium, pour préparer le dérivé cyanométhyle correspondant, suivie de l'hydrolyse du nitrile.

L'halogénation peut être effectuée dans un solvant chloré (dichlorométhane ou chloroforme par exemple), à une température comprise entre 0°C et la température de reflux du solvant.

Selon l'invention, les produits de formule générale (I) peuvent également être obtenus selon le procédé B, par condensation de la chaîne R₃ sur le dérivé de quinolyl propyl pipéridine (II') de formule générale : 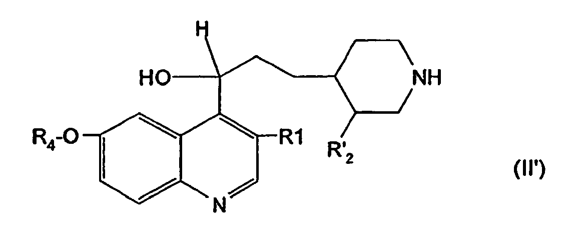 dans laquelle R₁ et R₄ sont définis comme précédemment et R'₂ représente un radical carboxy ou carboxyméthyle protégé, pour obtenir un dérivé de quinolyl propyl pipéridine de formule générale (III'): 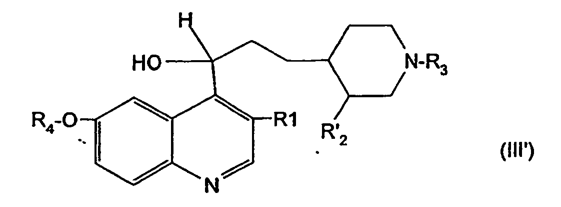 dans laquelle R₁, R'₂ et R₄ sont définis comme ci-dessus et R₃ est défini comme précédemment, puis oxydation en cétone de la fonction alcool en position alpha de la quinoléine pour obtenir un dérivé de formule (III) telle que définie précédemment, dans laquelle R₁, R'₂ et R₄ sont définis comme précédemment et K représente un atome d'oxygène, et poursuite de la synthèse comme décrit précédemment.

La condensation de la chaîne R₃ sur la pipéridine est effectuée dans les mêmes conditions que celles décrites dans la méthode A.

L'oxydation en cétone de la fonction alcool est effectuée par les méthodes classiques n'altérant pas le reste de la molécule, par exemple par oxydation selon D.Swern, J.O.C., 44, 41-48 (1979), notamment en présence de chlorure d'oxalyle et de diméthylsulfoxyde, le cas échéant dans un solvant, par exemple le dichlorométhane, à un température comprise entre -60 et 20°C.

Lorsque R₁ est un hydrogène, l'intermédiaire (II) ainsi que l'intermédiaire (II') peuvent être préparés selon une méthode décrite dans le brevet FR 99 11679.

Le dérivé de quinolyl propyl pipéridine de formule générale (II), pour lequel R₁ est un atome de fluor peut être préparé par oxydation du dérivé de quinolyl propyl pipéridine de formule générale (V) _{:} 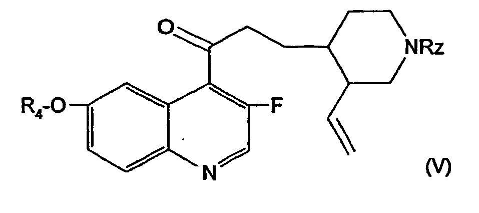 dans lequel Rz représente un radical protecteur d'amino, protection éventuelle du radical carboxy ainsi obtenu, puis, le cas échéant, après protection de la cétone, réduction du radical carboxy où du radical carboxy protégé en un radical hydroxyméthyle et transformation de celui-ci en un radical carboxyméthyle comme décrit plus haut, protection de ce dernier et libération de la cétone et de l'amine.

L'oxydation du dérivé de formule (V) est faite de préférence en deux étapes : on obtient tout d'abord le diol par oxydation du groupement vinyl par l'osmate de potassium dihydraté et le N-oxyde de 4-méthyl morpholine dans un mélange de dichlorométhane et d'eau à une température comprise entre 0°C et la température de reflux du solvant, de préférence à 30°C, puis on oxyde le diol par le permanganate de potassium et le métaperiodate de sodium dans un mélange d'acétonitrile et d'eau à une température comprise entre 0°C et la température de reflux du solvant, de préférence à 0°C. Les protections et déprotections du carboxy, de la cétone et de l'amine sont réalisées par les méthodes habituelles, rappelées le cas échéant plus haut.

La réduction du carboxy ou du carboxy protégé ainsi que la transformation de l'hydroxyméthyle en carboxyméthyle sont également réalisées par les méthodes rappelées plus haut.

Selon l'invention, le dérivé de quinolyl propyl pipéridine de formule générale (V) est obtenu par condensation du dérivé lithié en position 4 de la quinoléine (VI) : 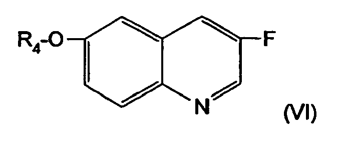 sur un dérivé de pipéridine de formule générale(VII) : 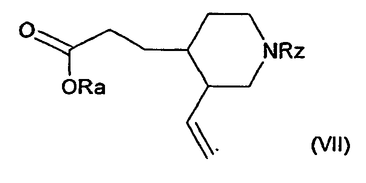 dans lequel Rz est un groupement protecteur d'amine défini précédemment et Ra est un groupement alkyle renfermant de 1 à 4 atomes de carbone, préférentiellement un méthyle.

La formation du dérivé lithié en position 4 de la quinoléine (VI) se fait à l'aide d'une base lithiée forte comme le butyllithium, le sec-butyllithium, ou de préférence le lithium diisopropylamidure, dans un solvant tel qu'un éther, le tétrahydrofurane par exemple, à une température comprise entre -78°C et -40°C. La condensation de ce dérivé lithié de quinoléine sur l'ester (VII) se fait dans le même solvant, à une température comprise entre -78°C et 0°C.

Le dérivé de quinoléine (VI) peut être préparé selon la méthode décrite dans la demande de brevet WO200240474.

Le dérivé de pipéridine de formule générale (VII) peut être préparé par réarrangement de Beckmann de l'oxime (VIII) 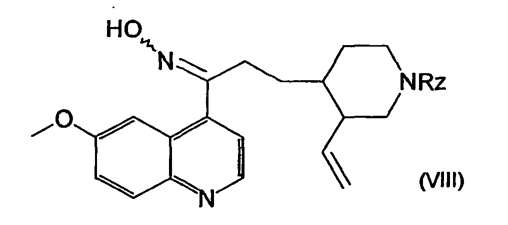 suivi du clivage de l'amide ainsi obtenu en acide, puis estérification de cet acide par les méthodes classiques décrites de la littérature.

Le réarrangement de Beckmann de l'oxime (VIII) en amide peut être réalisé selon les méthodes décrites dans la littérature (M. Smith, J.March, Advanced Organic Chemistry, 5ème Edition, p1415) par exemple par réaction d'un chlorure d'acide sulfonique (comme le chlorure de paratoluène sulfonyle) sur l'oxime dans un solvant comme l'acétone aqueuse ou le dichlorométhane en présence d'une base telle que la soude ou la potasse aqueuse ou une amine comme la triéthylamine ou Ia diisopropyléthylamine par exemple, à une température comprise entre 20°C et la température de reflux du solvant.

Le clivage de l'amide produit par le réarrangement de Beckmann en acide peut être réalisé selon les méthodes classiques de la littérature, comme l'hydrolyse basique par la soude ou la potasse, ou bien encore par exemple par traitement de l'amide par le dicarbonate de di-tert-butyle dans le dichlorométhane en présence de triéthylamine et de 4-diméthylamino-pyridine suivi d'un traitement par l'hydroxyde de lithium monohydraté et une solution aqueuse de peroxyde d'hydrogène en utilisant comme solvant le tétrahydrofurane par exemple.

L'oxime (VIII) peut être obtenue à partir du dérivé de quinolyl propyl pipéridine de formule générale (IX) : 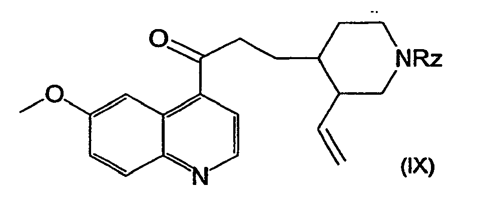 par réaction avec le chlorhydrate d'hydroxylamine dans la pyridine ou bien dans un mélange d'eau et d'alcool comme le méthanol en présence d'une base comme l'acétate de sodium par exemple.

Le dérivé de quinolyl propyl pipéridine de formule générale (IX) peut être préparé par application de la méthode décrite dans la demande de brevet FR 2354771.

Lorsque R₁ est un fluor et R'₂ est un carboxyméthyle, le dérivé de quinolyl propyl pipéridine de formule générale (II') peut être préparé par oxydation en milieu basique du dérivé de quinolyl propyl pipéridine de formule générale (X) : 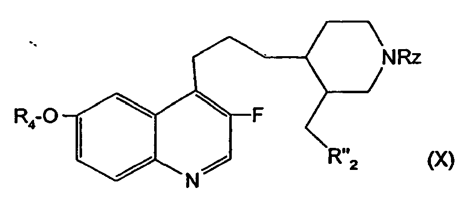 dans laquelle R₄ est défini comme précédemment, Rz est un groupement protecteur de l'amino, et R"₂ est le radical carboxy protégé correspondant à R'₂, puis déprotection de l'amino. L'oxydation s'effectué par action de l'oxygène, de préférence au sein d'un solvant inerte tel que le diméthylsulfoxyde, en présence de tert-butanol et d'une base telle que le tert-butylate de potassium ou de sodium, à une température comprise entre 0 et 100°C. La déprotection de la fonction amine de la pipéridine se fait selon les méthodes classiques rappelées plus haut.

Le dérivé de quinolyl propyl pipéridine de formule générale (X) peut être préparé par hydrogénation sélective du dérivé de quinolyl propyl pipéridine de formule générale(XI) : 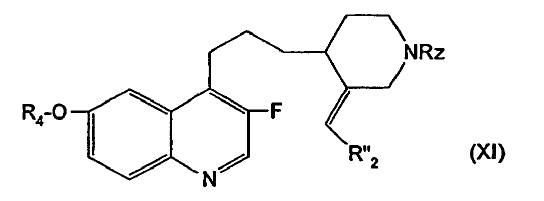 dans laquelle R₄, Rz et R"₂ sont définis comme ci-dessus, sous une pression de 1 à 100 bars et à une température comprise entre 20 et 80°C, dans un solvant tel qu'un alcool, éthanol par exemple, ou un amide diméthylformamide par exemple en présence d'un catalyseur, par exemple le palladium sur charbon ou le palladium sur sulfate de baryum. Le radical protecteur est plus particulièrement le radical benzyloxycarbonyle. Dans ce cas, la réaction d'hydrogénation conduit directement à la déprotection de l'amine.

Le dérivé de quinolyl propyl pipéridine de formule générale (XI) peut être préparé par condensation d'un dérivé de quinoléine de formule générale (XII) : 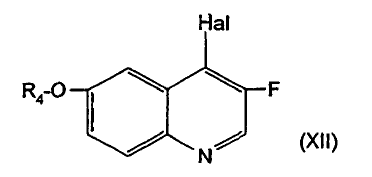 dans laquelle R₄ est définis comme précédemment et Hal représente un atome d'iode ou de brome, sur un dérivé de la pipéridine de formule générale (XIII): 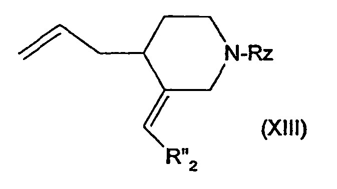 dans laquelle R"₂ et Rz sont définis comme ci-dessus.

La réaction s'effectue par action successive d'un organoborane (9-borabicyclo[3,3,1]nonane par exemple) dans un solvant tel qu'un éther (tétrahydrofurane, dioxane par exemple) à une température comprise entre -20 et 20°C, puis de l'addition du dérivé de quinoléine de formule générale (VII), par analogie avec les méthodes décrites par Suzuki et al., Pure and Appl. Chem., 57, 1749 (1985). La réaction s'effectue généralement en présence d'un sel de palladium (chlorure de palladium diphénylphosphinoferrocène par exemple) et d'une base comme le phosphate de potassium à une température comprise entre 20°C et la température de reflux du solvant.

Le dérivé de la pipéridine de formule générale (XIII) peut être préparé par réaction de Wittig, par condensation d'un ylure de phosphore sur un dérivé de pipéridine de formule générale (XIV) : 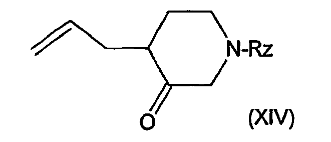 dans laquelle Rz est défini comme ci-dessus.

On opère avantageusement au moyen de (triphénylphosphorany-lidène) acétate de méthyle, dans un solvant comme par exemple le toluène, à une température comprise entre 20 et 110°C.

Le dérivé d'oxo-3 pipéridine de formule générale (XIV) peut être préparé selon ou par analogie avec la méthode décrite par Y. Takeuchi et coll., Synthesis, 10, 1814 (1999).

Le dérivé de quinoléine de formule générale (XII) dans laquelle R₁ représente un atome de fluor peut être préparé selon la méthode décrite dans le brevet WO200240474-A2.

Le dérivé de quinolyl propyl pipéridine de formule générale (II'), pour lequel R'₂ est un radical carboxy protégé peut être préparé à partir du dérivé correspondant pour lequel R'₂ est un radical carboxyméthyle protégé, l'hydroxy en alpha de la quinoléine étant protégé, par réduction de ce radical en alcool, transformation en un dérivé p-toluènesulfonyloxy, éthyle puis transformation de ce dérivé en dérivé vinylique par réaction d'élimination suivie de l'oxydation du dérivé obtenu, puis de la déprotection de la fonction alcool en alpha de la quinoléine et de la protection du radical carboxy.

La réduction de l'acide protégé en un radical hydroxyéthyle s'effectue selon les méthodes habituelles qui n'altèrent pas le reste de la molécule, notamment on opère par action d'un hydrure, hydrure d'aluminium et de lithium ou hydrure de diisobutyl aluminium par exemple, dans un solvant tel qu'un éther, tétrahydrofurane par exemple, à une température comprise entre 20 et 60°C.

La transformation du dérivé hydroxyéthyle en un dérivé p.toluènesulfonyloxyéthyle s'effectue notamment selon la méthode décrite par L.F. Fieser et M. Fieser, Reagents for Organic Synthesis, vol 1, 1179 (1967), à partir du chlorure de p-toluènesulfonyle en présence d'une base comme une amine tertiaire, par exemple la triéthylamine, ou aromatique, par exemple la pyridine, dans un solvant halogéné, dichlorométhane par exemple, ou sans solvant, à une température comprise entre 0 et 50°C.

La transformation du dérivé p-toluènesulfonyloxyéthyle en dérivé vinylique s'effectue par réaction d'élimination, notamment selon la méthode décrite par A. Sharma et coll., Org. Prep Proced. Int., 25(3), 330-333 (1993), en présence d'une base, par exemple le t-butylate de potassium, dans un solvant, le diméthylsulfoxyde par exemple, à une température comprise entre 20 et 100°C.

La transformation du dérivé vinylique en un dérivé carboxy s'effectue par les méthodes d'oxydation décrites dans la littérature, notamment par le méta periodate de sodium en présence d'hydrate de trichlorure de ruthénium, dans un mélange de solvants comme par exemple le mélange eau/acétonitrile, à une température comprise entre 20 et 60°C.

Il est entendu que les dérivés de formule générale (I), mais aussi les intermédiaires de formules (II) et (II'), (III) et (III'), (V), (VIII), (IX), (X) ainsi que leurs intermédiaires de préparation présentent une isomérie «cis/trans» au niveau des substituants en position 3 et 4 de la pipéridine. Les dérivés de configuration «trans» peuvent être obtenus à partir des dérivés de configuration «cis» selon ou par analogie avec la méthode décrite dans la demande internationale WO 99/37635, ou à partir d'intermédiaires existant sous forme de mélanges, après séparation selon les méthodes connues.

Les dérivés de quinolyl propyl pipéridine de formule générale (I) peuvent être purifiés, le cas échéant, par des méthodes physiques telles que la cristallisation ou la chromatographie.

Par ailleurs, il est également entendu que les composés de formule générale (I) existent également sous formes énantiomères et diastéréoisomères, lesquelles formes ainsi que leurs mélanges entrent dans le cadre de la présente invention. Ces derniers peuvent la cas échéant être séparés notamment par chromatographie sur silice ou par Chromatographie Liquide Haute Performance (CLHP). De même, les dérivés cis et trans peuvent être séparés par chromatographie sur silice ou par Chromatographie Liquide Haute Performance (CLHP).

Les dérivés de quinolyl propyl pipéridine de formule générale (I) peuvent être transformés en sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Certains des dérivés de quinolyl propyl pipéridine de formule générale (I) portant un radical carboxy peuvent être transformés à l'état de sels métalliques ou en sels d'addition avec les bases azotées selon les méthodes connues en soi. Ces sels entrent également dans le cadre de la présente invention. Les sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-□-phénéthylamine, NN'-dibenzyléthylènediamine, diphénylènediamine, benzhydrylamine, quinine, choline, arginine, lysine, leucine, dibenzylamine).

Les dérivés de quinolyl propyl pipéridine selon l'invention sont des agents antibactériens particulièrement intéressants.

In vitro, sur germes gram, positifs les dérivés de quinolyl propyl pipéridine selon l'invention se sont montrés actifs à des concentrations comprises entre 0,03 et 4 µg/ml sur *Staphylococcus aureus* AS5155 résistante à la méticilline, également à des concentrations comprises entre 0,06 et 8 µg/ml sur *Streptococcus pneumoniae* 6254-01 et à des concentrations comprises entre 0,06 et 64 µg/ml sur *Enterococcus faecium* H983401 et, sur germes gram négatifs, ils se sont montrés actifs à des concentrations comprises entre 0,12 et 32 µg/ml sur *Moraxella catharrhalis* IPA152 ; in vivo, ils se sont montrés actifs sur les infections expérimentales de la souris à *Staphylococcus* aureus IP8203 à des doses comprises entre 12 et 150 mg/kg par voie sous cutanée (DC₅₀) et pour certains d'entre eux à des doses comprises entre 26 et 150 mg/kg par voie orale.

Enfin, les produits selon l'invention sont particulièrement intéressants du fait de leur faible toxicité. Aucun des produits n'a manifesté de toxicité à la dose de 100 mg/kg par voie sous cutanée chez la souris.

Ces propriétés rendent aptes lesdits produits, ainsi que leurs sels d'acides et de bases pharmaceutiquement acceptables, à être utilisés comme médicaments dans le traitement des affections à germes sensibles provoquées par des bactéries à gram (+) et notamment dans celui des staphylococcies, telles que septicémies à staphylocoques, staphylococcies malignes de la face ou cutanée, pyodermites, plaies septiques ou suppurantes, anthrax, phlegmons, érysipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

La présente invention a donc également pour objet, à titre de médicaments et notamment de médicaments destinés au traitement des infections bactériennes chez l'homme ou l'animal, les composés de formule (I) tels que définis ci-dessus ainsi que leurs sels pharmaceutiquement acceptables, et notamment les composés préférés mentionnés plus haut.

La présente invention concerne également les compositions pharmaceutiques contenant au moins un dérivé de quinolyl propyl pipéridine selon l'invention, le cas échéant sous forme de sel, à l'état pur ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions pour administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de quinolyl propyl pipéridine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 750 mg et 3 g de produit actif en 2 ou 3 prises par jour par voie orale ou entre 400 mg et 1,2 g par voie intraveineuse pour un adulte.

L'exemple suivant illustre une composition selon l'invention.

On prépare selon la technique habituelle une composition liquide destinée à l'usage parentéral comprenant :
- Acide (3R,4R) -4-[3-(3-fluoro-6-méthoxyquinolin-4-yl)-3-oxopropyl]-1-[3-(2,3,5-trifluorophénylthio)prop-2-ynyl] pipéridine-3-carboxylique..... 1 g
- Glucose..... qsp 2,5%
- hydroxyde de sodium..... qsp pH = 4-4,5
- eau ppi..... qsp 20 ml

L'invention a enfin pour objet, à titre de produits industriels nouveaux et notamment à titre de produits intermédiaires nécessaires à la préparation des produits de formule (I) :
- les produits de formule (II) telle que définie plus haut, dans laquelle R₁ est un atome de fluor et la cétone est libre ou protégée ;
- les produits de formule (A) : 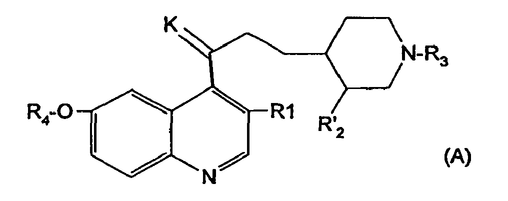 dans laquelle R₁, R'₂, R₃ et R₄ sont tels que définis précédemment et K représente un groupement protecteur de cétone ;
- les produits de formule (B) : 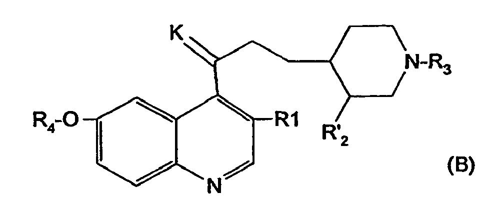 dans laquelle R₁, R₂, R₃ et R₄ sont tels que définis précédemment et K représente un groupement protecteur de cétone, correspondant à des produits obtenus à l'issue de différents traitements effectués sur les produits de formule (III) ;
- les produits de formule (V) telle que définie plus haut ;
- Les produits de formule (C) : 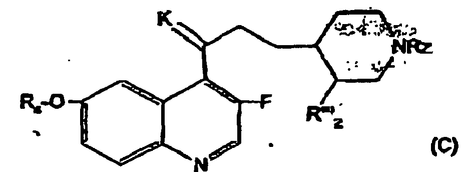 dans laquelle R₄, Rz et K sont tels que définis précédemment et R'''₂ représente un radical carboxy ou carboxyméthyle libre ou protégé ou un radical hydroxyméthyle, correspondant à des produits obtenus à l'issue des différents traitements effectués sur les produits de formule (V) ;
- les produits de formule (VIII) telle que définie plus haut.

Parmi les produits selon l'invention, plus particulièrement intéressants sont les dérivés de quinolyl propyl pipéridine cités ci-après, et notamment ceux décrits dans les exemples, à titre non limitatif :
- Acide (3BS,4RS) ou (3SR,4RS)-4-[3-oxo-3-(6-méthoxyquinolin-4-yl)propyl]-1-(2-phénylthioéthyl)pipéridine-3-carboxylique
- Acide (3RS,4RS) ou (3SR, 4RS)-4-[3-oxo-3-(6-méthoxyquinolin-4-yl)propyl]-1-[2-(3-fluorophénylthio)éthyl]pipéridine-3-carboxylique
- Acide (3RS,4RS) ou (3SR,4RS)-4-[3-oxo-3-(6-méthoxyquinolin-4-yl)propyl]-1-[2-(2,5-difluorophénylthio)éthyl)pipéridine-3-carboxylique
- Acide (3RS,4RS) ou (3SR,4R5)-4-[3-oxo-3-(6-méthoxyquinolin-4-yl)propyl]-1-[2-(3,5-difluorophénylthio)éthyl]pipéridine-3-carboxylique
- Acide (3RS,4RS) ou (3SR,4RS)-4-[3-oxo-3-(6-méthoxyquinolin-4-yl)propyl]-1-[2-(2,3,5-trifluorophénylthio)éthyl]pipéridine-3-carboxylique
- Acide (3RS,4RS) ou (3SR,4RS)-4-[3-oxo-3-(6-méthoxyquinolin-4-yl)propyl]-1-[2-(n-propylthio)éthyl]pipéridine-3-carboxylique
- Acide (3RS,4RS) ou (3SR,4RS)-4-[3-oxo-3-(6-méthoxyquinolin-4-yl)propyl]-1-[2-(n-butylthio)éthyl]pipéridine-3-carboxylique
- Acide (3RS,4RS) ou (3SR,4RS)-4-[3-oxo-3-(6-méthoxyquinolin-4-yl)propyl]-1-[2-(cyclopropylthio)éthyl]pipéridine-3-carboxylique
- Acide (3RS, 4RS) ou (3SR,4RS)-4-[3-oxo-3-(6-méthoxyquinolin-4-yl)propyl]-1-[2-(cyclobutylthio)éthyl]pipéridine-3-carboxylique
- Acide (3RS,4RS) ou (3SR,4RS)-4-[3-oxo-3-(6-méthoxyquinolin-4-yl)propyl]-1-[2-(cyclopentylthio)éthyl]pipéridine-3-carboxylique
- Acide (3RS,4RS) ou (3SR,4RS)-4-[3-oxo-3-(6-méthoxyquinolin-4-yl)propyl]-1-[2-(cyclohexylthio)éthyl]pipéridine-3-carboxylique
- Acide (3RS,4RS) ou (3SR,4RS)-4-[3-oxo-3-(6-méthoxyquinolin-4-yl)propyl]-1-[2-(thien-2-yl)thioéthyl]pipéridine-3-carboxylique
- Acide (3RS,4RS) ou (3SR,4RS)-4-[3-oxo-3-(6-méthoxyquinolin-4-yl)propyl]-1-[2-(5-fluorothien-2-yl)thioéthyl]pipéridine-3-carboxylique
- Acide (3RS,4RS) ou (3SR,4RS)-4-[3-oxo-3-(6-méthoxyquinolin-4-yl)propyl]-1-[2-(4-fluorothien-2-yl)thioéthyl]pipéridine-3-carboxylique
- Acide (3RS,4RS) ou (3SR,4RS)-4-[3-oxo-3-(6-méthoxyquinolin-4-yl)propyl]-1-[2-(3-fluorothien-2-yl)thioéthyl]pipéridine-3-carboxylique
- Acide (3RS,4RS) ou (3SR,4RS)-4-[3-oxo-3-(6-méthoxyquinolin-4-yl)propyl]-1-[2-(thien-3-yl)thioéthyl]pipéridine-3-carboxylique
- Acide (3RS,4RS) ou (3SR,4RS)-4-[3-oxo-3-(6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-oxo-3-(6-méthoxyquinolin-4-yl)propyl]-1-[2-(pyridin-2-yl)thioéthyl]pipéridine-3-carboxylique
- Acide (3RS,4RS) ou (3SR,4RS)-4-[3-oxo-3-(6-méthoxyquinolin-4-yl)propyl]-1-[2-(4-fluoropyridin-2-yl)thioéthyl]pipéridine-3-carboxylique
- Acide (3RS,4RS) ou (3SR,4RS)-4-[3-oxo-3-(6-méthoxyquinolin-4-yl)propyl]-1-[2-(3-fluoro-pyridin-2-yl)thioéthyl]pipéridine-3-carboxylique
- Acide (3RS,4RS) ou (3SR,4RS)-4-[3-oxo-3-(6-méthoxyquinolin-4-yl)propyl]-1-[3-(3-fluoro-phényl)-prop-2-ynyl]-pipéridine-3-carboxylique
- Acide (3RS,4RS) ou (3SR,4RS)-4-[3-oxo-3-(6-méthoxyquinolin-4-yl)propyl]-1-[3-(2,5-difluoro-phényl)-prop-2-ynyl]-pipéridine-3-carboxylique
- Acide (3RS,4RS) ou (3SR,4RS)-4-[3-oxo-3-(6-méthoxyquinolin-4-yl)propyl]-1-[3-(3,5-difluoro-phényl)-prop-2-ynyl]-pipéridine-3-carboxylique
- Acide (3RS,4RS) ou (3SR,4RS)-4-[3-oxo-3-(6-méthoxyquinolin-4-yl)propyl]-1-[3-(2,3,5-trifluoro-phényl)-prop-2-ynyl]-pipéridine-3-carboxylique
- Acide (3RS,4RS) ou (3SR,4RS)-4-[3-oxo-3-(6-méthoxyquinolin-4-yl)propyl]-1-[3-(thien-2-yl)-prop-2-ynyl]-pipéridine-3-carboxylique
- Acide (3RS,4RS) ou (3SR,4RS)-43-oxo-3-(6-méthoxyquinolin-4-yl)propyl]-1-[3-(5-fluorothien-2-yl)-prop-2-ynyl]-pipéridine-3-carboxylique
- Acide (3RS,4RS) ou (3SR,4RS)-4-[3-oxo-3-(6-méthoxyquinolin-4-yl)propyl]-1-[3-(4-fluorothien-2-yl)-prop-2-ynyl]-pipéridine-3-carboxylique
- Acide (3RS,4RS) ou (3SR,4RS)-4-[3-oxo-3-(6-méthoxyquinolin-4-yl)propyl]-1-[3-(3-fluorothien-2-yl)-prop-2-ynyl]-pipéridine-3-carboxylique
- Acide (3RS,4RS) ou (3SR,4RS)-4-[3-oxo-3-(3-fluoro-6-méthoxyquinolin-4-yl)propyl]-1-(2-phénylthioéthyl)pipéridine-3-carboxylique
- Acide (3RS,4RS) ou (3SR,4RS)-4-[3-oxo-3-(3-fluoro-6-méthoxyquinolin-4-yl)propyl]-1-[2-(3-fluorophénylthio)éthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(2,5-difluorophénylthio) éthyl] pipéridine-3-carboxylique
- Acide (3RS,4RS) ou (3SR,4RS)-4-[3-oxo-3-(3-fluoro-6-méthoxyquinolin-4-yl)propyl]-1-[2-(3,5-difluorophénylthio)éthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(2,3,5-trifluorophénylthio)éthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(n-propylthio)éthylpipéridine-3-carboxylique
- Acide (3RS,4RS) ou (3SR,4RS)-4-[3-oxo-3-(3-fluoro-6-méthoxyquinolin-4-yl)propyl]-1-[2-(n-butylthio)éthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(cyclopropylthio)éthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(cyclobutylthio)éthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(cyclopentylthio)éthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(cyclohexylthio)éthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(thien-2-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(5-fluorothien-2-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(4-fluorothien-2-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(3-fluorothien-2-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(thien-3-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(pyridin-2-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(4-fluoropyridin-2-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[2-(3-fluoro-pyridin-2-yl)thioéthyl]pipéridine-3-carboxylique
- Acide (3RS,4RS) ou (3SR,4RS)-4-[3-oxo-3-(3-fluoro-6-méthoxyquinolin-4-yl)propyl]-1-[3-(3-fluoro-phényl)-prop-2-ynyl]-pipéridine-3-carboxylique
- Acide (3RS,4RS) ou (3SR,4RS)-4-[3-oxo-3-(3-fluoro-6-méthoxyquinolin-4-yl)propyl]-1-[3-(2,5-difluoro-phényl)-prop-2-ynyl]-pipéridine-3-carboxylique
- Acide (3RS,4RS) ou (3SR,4RS)-4-[3-oxo-3-(3-fluoro-6-méthoxyquinolin-4-yl)propyl]-1-[3-(3,5-difluoro-phényl)-prop-2-ynyl]-pipéridine-3-carboxylique
- Acide (3RS,4RS) ou (3SR,4RS)-4-[3-oxo-3-(3-fluoro-6-méthoxyquinolin-4-yl)propyl]-1-[3-(2,3,5-trifluoro-phényl)-prop-2-ynyl]-pipéridine-3-carboxylique
- Acide (3RS,4RS) ou (3SR,4RS)-4-[3-oxo-3-(3-fluoro-6-méthoxyquinolin-4-yl)propyl]-1-[3-(thien-2-yl)-prop-2-ynyl]-pipéridine-3-carboxylique
- Acide (3RS,4RS) ou (3SR,4RS)-43-oxo-3-(3-fluoro-6-méthoxyquinolin-4-yl)propyl]-1-[3-(5-fluorothien-2-yl)-prop-2-ynyl]-pipéridine-3-carboxylique
- Acide (3RS, 4RS) ou (3SR,4RS)-4-[3-oxo-3-(3-fluoro-6-méthoxyquinolin-4-yl)propyl]-1-[3-(4-fluorothien-2-yl)-prop-2-ynyl]-pipéridine-3-carboxylique
- Acide (3RS,4RS) ou (3SR,4RS)-4-[3-oxo-3-(3-fluoro-6-méthoxyquinolin-4-yl)propyl]-1-[3-(3-fluorothien-2-yl)-prop-2-ynyl]-pipéridine-3-carboxylique
- Acide (3RS,4RS) où (3SR,4RS)-4-[3-oxo-3-(3-fluoro-6-méthoxyquinolin-4-yl)propyl]-1-[3-(thien-3-yl)-prop-2-ynyl]-pipéridine-3-carboxylique
- Acide (3RS, 4RS) ou (3SR,4RS)-4-[3-oxo-3-(6-méthoxyquinolin-4-yl)propyl]-1-(2-phénylthioéthyl)pipéridine-3-acétique
- Acide (3RS,4RS) ou (3SR,4RS)-4-[3-oxo-3-(6-méthoxyquinolin-4-yl)propyl]-1-[2-(3-fluorophénylthio)éthyl]pipéridine-3-acétique
- Acide (3RS,4RS) ou (3SR,4RS)-4-[3-oxo-3-(6-méthoxyquinolin-4-yl)propyl]-1-[2-(2,5-difluorophénylthio)éthyl]pipéridine-3-acétique
- Acide (3RS,4RS) ou (3SR,4RS)-4-[3-oxo-3-(6-méthoxyquinolin-4-yl)propyl]-1-[2-(3,5-difluorophénylthio)éthyl]pipéridine-3-acétique
- Acide (3RS,4RS) ou (3SR,4RS)-4-[3-oxo-3-(6-méthoxyquinolin-4-yl)propyl]-1-[2-(2,3,5-trifluorophénylthio)éthyl]pipéridine-3-acétique
- Acide (3RS,4RS) ou (3SR,4RS)-4-[3-oxo-3-(6-méthoxyquinolin-4-yl)propyl)-1-[2-(n-propylthio)éthyl]pipéridine-3-acétique
- Acide (3RS,4RS) ou (3SR,4RS)-4-[3-oxo-3-(6-méthoxyquinolin-4-yl)propyl]-1-[2-(n-butylthio)éthyl]pipéridine-3-acétique
- Acide (3RS,4RS) ou (3SR,4RS)-4-[3-oxo-3-(6-méthoxyquinolin-4-yl)propyl]-1-[2-(cyclopropylthio)éthyl]pipéridine-3-acétique
- Acide (3RS,4RS) ou (3SR,4RS)-4-[3-oxo-3-(6-méthoxyquinolin-4-yl)propyl]-1-[2-(cyclobutylthio)éthyl]pipéridine-3-acétique
- Acide (3RS,4RS) ou (3SR,4RS)-4-[3-oxo-3-(6-méthoxyquinolin-4-yl)propyl]-1-[2-(cyclopentylthio)éthyl]pipéridine-3-acétique
- Acide (3RS,4RS) ou (3SR,4RS)-4-[3-oxo-3-(6-méthoxyquinolin-4-yl)propyl]-1-[2-(cyclohexylthio)éthyl]pipéridine-3-acétique
- Acide (3RS,4RS) ou (3SR,4RS)-4-[3-oxo-3-(6-méthoxyquinolin-4-yl)propyl]-1-[2-(thien-2-yl)thioéthyl]pipéridine-3-acétique
- Acide (3RS,4RS) ou (3SR,4RS)-4-[3-oxo-3-(6-méthoxyquinolin-4-yl)propyl]-1-[2-(5-fluorothien-2-yl)thioéthyl]pipéridine-3-acétique
- Acide (3RS,4RS) ou (3SR,4RS)-4-[3-oxo-3-(6-méthoxyquinolin-4-yl)propyl]-1-[2-(4-fluorothien-2-yl)thioéthyl]pipéridine-3-acétique
- Acide (3RS,4RS) ou (3SR,4RS)-4-[3-oxo-3-(6-méthoxyquinolin-4-yl)propyl]-1-[2-(3-fluorothien-2-yl)thioéthyl]pipéridine-3-acétique
- Acide (3RS,4RS) ou (3SR,4RS)-4-[3-oxo-3-(6-méthoxyquinolin-4-yl)propyl]-1-[2-(thien-3-yl)thioéthyl]pipéridine-3-acétique
- Acide (3RS,4RS) ou (3SR,4RS)-4-[3-oxo-3-(6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-oxo-3-(6-méthoxyquinolin-4-yl)propyl]-1-[2-(pyridin-2-yl)thioéthyl]pipéridine-3-acétique
- Acide (3RS,4RS) ou (3SR,4RS)-4-[3-oxo-3-(6-méthoxyquinolin-4-yl)propyl]-1-[2-(4-fluoropyridin-2-yl)thioéthyl]pipéridine-3-acétique
- Acide (3RS,4RS) ou (3SR,4RS)-4-[3-oxo-3-(6-méthoxyquinolin-4-yl)propyl]-1-[2-(3-fluoro-pyridin-2-yl)thioéthyl]pipéridine-3-acétique
- Acide (3RS,4RS) ou (3SR, 4RS)-4-[3-oxo-3-(6-méthoxyquinolin-4-yl)propyl]-1-[3-(3-fluoro-phényl)-prop-2-ynyl]-pipéridine-3-acétique
- Acide (3RS,4RS) ou (3SR,4RS)-4-[3-oxo-3-(6-méthoxyquinolin-4-yl)propyl]-1-[3-(2,5-difluoro-phényl)-prop-2-ynyl]-pipéridine-3-acétique
- Acide (3RS,4RS) ou (3SR,4RS)-4-[3-oxo-3-(6-méthoxyquinolin-4-yl)propyl]-1-[3-(3,5-difluoro-phényl)-prop-2-ynyl]-pipéridine-3-acétique
- Acide (3RS,4RS) ou (3SR,4RS)-4-[3-oxo-3-(6-méthoxyquinolin-4-yl)propyl]-1-[3-(2,3,5-trifluoro-phényl)-prop-2-ynyl]-pipéridine-3-acétique
- Acide (3RS,4RS) ou (3SR,4RS)-4-[3-oxo-3-(6-méthoxyquinolin-4-yl)propyl]-1-[3-(thien-2-yl)-prop-2-ynyl]-pipéridine-3-acétique
- Acide (3RS,4RS) ou (3SR,4RS)-43-oxo-3-(6-méthoxyquinolin-4-yl)propyl]-1-[3-(5-fluorothien-2-yl)-prop-2-ynyl]-pipéridine-3-acétique
- Acide (3RS,4RS) ou (3SR,4RS)-4-[3-oxo-3-(6-méthoxyquinolin-4-yl)propyl]-1-[3-(4-fluorothien-2-yl)-prop-2-ynyl]-pipéridine-3-acétique
- Acide (3RS,4RS) ou (3SR,4RS)-4-[3-oxo-3-(6-méthoxyquinolin-4-yl)propyl]-1-[3-(3-fluorothien-2-yl)-prop-2-ynyl]-pipéridine-3-acétique
- Acide (3RS,4RS) ou (3SR,4RS)-4-[3-oxo-3-(6-méthoxyquinolin-4-yl)propyl]-1-[3-(thien-3-yl)-prop-2-ynyl]-pipéridine-3-acétique

### Exemple 1

### Acide (3R,4R)-1-(2-cyclohexylsulfanyl-éthyl)-4-[3-(3-fluoro-6-méthoxy-quinolin-4-yl)-3-oxo-propyl]-pipéridine-3-carboxylique

Une solution de 0,084 g (0,163 mmol) de (3R,4R)-1-(2-cyclohexylsulfanyl-éthyl)-4-[3-(3-fluoro-6-méthoxy-quinolin-4-yl)-3-oxo-propyl]-pipéridine-3-carboxylate de méthyle dans 4,5 cm³ d'acide chlorhydrique 5N est chauffée à une température voisine de 80°C pendant 5 heures. Après arrêt du chauffage puis retour à température ambiante, on ajoute successivement 10 cm³ de CH₂Cl₂ et 5 cm³ d'eau. On ajoute alors une solution aqueuse d'hydroxyde de sodium 5N puis 1N afin d'ajuster le pH de la phase aqueuse à une valeur voisine de 8. La phase organique est décantée puis la phase aqueuse est extraite avec 2 fois 15 cm³ de CH₂Cl₂. Les phases organiques sont réunies, séchées sur sulfate de magnésium anhydre, filtrées et concentrées à sec sous pression réduite (2,7 kPa) pour donner 0,073 g d'une huile orange qui est purifiée par chromatographie-flash [éluant : dichlorométhane / méthanol / acétonitrile (92 / 4 / 4 en volumes)]. Après concentration des fractions sous pression réduite, on obtient 0,03 g d'acide (3R,4R)-1-(2-cyclohexylsulfanyl-éthyl)-4-[3-(3-fluoro-6-méthoxy-quinolin-4-yl)-3-oxo-propyl]-pipéridine-3-carboxylique, sous forme d'une huile jaune.

Spectre de R.M.N. ¹H (300 MHz, (CD₃)₂SO d6, δ en ppm) : 1,25 (mt : 5H) ; de 1, 50 à 1,98 (mt : 10H) ; 2,28 (mt : 1H) ; 2,42 (d, J = 12 Hz : 1H) ; de 2,50 à 2,80 (mt : 6H) ; 2,88 (mt : 1H) ; 3,06 (t large: 1H) ; 3,15 (mt : 2H) ; 3,92 (s : 3H) ; 7,10 (d, J = 3 Hz : 1H) ; 7,50 (dd, J = 9 et 3 Hz : 1H) ; 8, 05 (d, J = 9 Hz : 1H) ; 8,90 (s : 1H) .

### Spectre de MS (IC) m/z 503 (M+H)+

Le (3R,4R)-1-(2-cyclohexylsulfanyl-éthyl)-4-[3-(3-fluoro-6-méthoxy-quinolin-4-yl)-3-oxo-propyl]-pipéridine-3-carboxylate de méthyle peut être préparé de la manière suivante :

A 1,5 g (4 mmol) de (3R,4R)-4-[3-(3-fluoro-6-méthoxy-quinolin-4-yl)-3-oxo-propyl]-pipéridine-3-carboxylate de méthyle en solution dans 20 cm³ d'acétonitrile et 0,8 cm³ de diméthylformamide, on ajoute à une température voisine de 20°C, sous atmosphère d'argon, 1,66 g de carbonate de potassium, 0,665 g d'iodure de potassium puis une solution de 0,738 g de 2-chloroéthyl cyclohexyl sulfure dans 10 cm³ d'acétonitrile. Après 16 heures d'agitation à reflux et refroidissement du mélange réactionnel, on ajoute 20 cm³ d'eau puis on concentre à sec sous pression réduite (2,7 kPa). Le résidu est repris avec 70 cm³ de CH₂Cl₂, lavé par 2 fois 35 cm³ d'eau puis 40 cm³ d'une solution aqueuse saturée en chlorure de sodium. La phase organique est séchée sur sulfate de magnésium anhydre, filtrée et concentrée à sec sous pression réduite (2,7 kPa) pour donner 2,2 g d'une huile orange qui est purifiée par chromatographie-flash [éluant : dichlorométhane / méthanol / acétonitrile (98 / 1 / 1 en volumes)]. Après concentration des fractions sous pression réduite, on obtient 0,67 g d'une huile orange qui est purifiée par chromatographie-flash [éluant : dichlorométhane/ méthanol / acétonitrile (98 / 2 / 2 en volumes)]. Après concentration des fractions sous pression réduite, on obtient 0,495 g de (3R,4R)-1-(2-cyclohexylsulfanyl-éthyl)-4-[3-(3-fluoro-6-méthoxy-quinolin-4-yl)-3-oxo-propyl]-pipéridine-3-carboxylate de méthyle, sous forme d'une huile orange.

### Spectre de MS (IE) m/z 516 (M+.) m/z 387 (pic de base)

Le (3R,4R)-4-[3-(3-fluoro-6-méthoxy-quinolin-4-yl)-3-oxo-propyl]-pipéridine-3-carboxylate de méthyle peut être préparé de la manière suivante :

A 4 g (7,23 mmol) de (3R, 4R)-1-*tert*-butyloxycarbonyl-4-[3-(3-fluoro-6-méthoxy-quinolin-4-yl)-3-oxo-propyl]-pipéridine-3-carboxylate de méthyle en solution dans 20 cm³ d'acétate d'éthyle, on ajoute à une température voisine de 20°C, 9 cm³ d'une solution 4 N d'acide chlorhydrique dans de l'acétate d'éthyle et 10 cm³ de MeOH. Après 4 heures d'agitation à une température voisine de 20°C, on dilue le mélange réactionnel avec 25 cm³ d'acétate d'éthyle puis on ajoute une solution aqueuse d'hydroxyde de sodium 5N afin d'ajuster le pH de la phase aqueuse à une valeur comprise entre 8 et 8,5. La phase organique est décantée puis la phase aqueuse est extraite avec 3 fois 40 cm³ d'acétate d'éthyle. Les phases organiques sont réunies, lavées avec 2 fois 50 cm³ d'une solution aqueuse saturée en chlorure de sodium, séchées sur sulfate de magnésium anhydre, filtrées et concentrées à sec sous pression réduite (2,7 kPa) pour donner 2,33 g de (3R,4R)-4-[3-(3-fluoro-6-méthoxy-quinolin-4-yl)-3-oxo-propyl]-pipéridine-3-carboxylate de méthyle, sous forme d'une huile orange.

### Spectre de MS (IE) m/z 374, (M+.)

Le (3R,4R)-1-*tert*-butyloxycarbonyl-4-[3-(3-fluoro-6-méthoxy-quinolin-4-yl)-3-oxo-propyl]-pipéridine-3-carboxylate de méthyle peut être préparé de la manière suivante :

A 3,33 g (7,23 mmol) d'acide (3R,4R)-1-tert-butyloxycarbonyl-4-[3-(3-fluoro-6-méthoxy-quinolin-4-yl)-3-oxo-propyl]-pipéridine-3-carboxylique en solution dans 150 cm³ de dichlorométhane, on ajoute à une température voisine de 20°C, sous atmosphère d'argon, 1,58 cm³ d'une solution de chlorure de thionyle. Après 1 heure d'agitation à une température voisine de 20°C, le mélange réactionnel est coulé sur une solution de 10,55 cm³ de N,N-diisopropyléthylamine dans 150 cm³ de méthanol. Après 16 heures d'agitation à une température voisine de 20°C, le mélange réactionnel est concentré à sec sous pression réduite (2,7 kPa). Le résidu est repris avec 150 cm³ de CH₂Cl₂, lavé par 3 fois 50 cm³ d'eau puis 50 cm³ d'une solution aqueuse saturée en chlorure de sodium. La phase organique est séchée sur sulfate de magnésium anhydre, filtrée et concentrée à sec sous pression réduite (2,7 kPa) pour donner 4 g de (3R,4R)-1-*tert*-butyloxycarbonyl-4- [3- (3-fluoro-6-méthoxy-quinolin-4-yl)-3-oxo-propyl]-pipéridine-3-carboxylate de méthyle, sous forme d'une huile orange.

### Spectre de MS (IC) m/z 475, (M+H)+

L'acide (3R,4R)-1-*tert*-butyloxycarbonyl-4-[3-(3-fluoro-6-méthoxy-quinolin-4-yl)-3-oxo-propyl]-pipéridine-3-carboxylique peut être préparé de la manière suivante :

A 3,7 g (7,76 mmol) de (3R,4R)-1-*tert*-butyloxycarbonyl-3-(1RS,2-dihydroxy-éthyl)-4-[3-(3-fluoro-6-méthoxy-quinolin-4-yl)-3-oxo-propyl]-pipéridine en solution dans 200 cm³ d'acétonitrile et 5 cm³ d'eau, on ajoute successivement à une température voisine de 0°C, sous atmosphère d'argon, 0,147 g de permanganate de potassium puis une solution de 4,15 g de métaperiodate de sodium dans 16 cm³ d'acétonitrile et 26 cm³ d'eau. Après 2 heures d'agitation à une température voisine de 0°C, on ajoute 100 cm³ d'une solution aqueuse saturée en sulfite de sodium. Après 2 heures d'agitation à une température voisine de 20°C, le milieu réactionnel est filtré sur Célite® sur verre fritté. La Célite® est rincée par 2 fois 20 cm³ d'acétonitrile. Le pH du filtrat est ajusté à une valeur comprise entre 4 et 5 par addition d'acide acétique. Le filtrat est ensuite concentré à sec sous pression réduite (2,7 kPa). Le résidu est repris avec 150 cm³ de CH₂Cl₂, lavé par 2 fois 50 cm³ d'eau puis 75 cm³ d'une solution aqueuse saturée en chlorure de sodium. La phase organique est séchée sur sulfate de magnésium anhydre, filtrée et concentrée à sec sous pression réduite (2,7 kPa) pour donner 3,33 g d'acide (3R,4R)-1-*tert*-butyloxycarbonyl-4-[3-(3-fluoro-6-méthoxy-quinolin-4-yl)-3-oxo-propyl]-pipéridine-3-carboxylique, sous forme d'une mousse jaune.

### Spectre de MS (IC) m/z 461, (M+H)+

Le (3R,4R)-1-*tert*-butyloxycarbonyl-3-(1RS,2-dihydroxy-éthyl)-4-[3-(3-fluoro-6-méthoxy-quinolin-4-yl)-3-oxo-propyl]-pipéridine peut être préparé de la manière suivante :

A 2,47 g (5, 02 mmol) de (3R,4R)-1-*tert*-butyloxycarbonyl-4-[3-(3-fluoro-6-méthoxy-quinolin-4-yl)-3-oxo-propyl]-3-vinyl-pipéridine en solution dans 56,2 cm³ de dichlorométhane et 1,25 cm³ d'eau, on ajoute successivement à une température voisine de 30°C, sous atmosphère d'argon, 1,3 g de N-oxyde de 4-méthyl morpholine puis 0,0562 g d'osmate de potassium dihydraté. Après 15 heures d'agitation à une température voisine de 30°C, on ajoute 100 cm³ d'une solution aqueuse saturée en sulfite de sodium. Après 10 minutes d'agitation à une température voisine de 20°C, la phase organique est décantée puis filtrée sur Célite® sur verre fritté. La Célite® est rincée par 2 fois 10 cm³ dichlorométhane. Le pH du filtrat est ajusté à une valeur voisine de 7 par addition d'acide acétique. Le filtrat est ensuite lavé par 2 fois 20 cm³ d'une solution aqueuse saturée en chlorure de sodium. La phase organique est séchée sur sulfate de magnésium anhydre, filtrée et concentrée à sec sous pression réduite (2,7 kPa) pour donner 3,1 g d'unè huile orange qui est purifiée par chromatographie-flash [éluant : dichlorométhane / méthanol / acétonitrile (98 / 2 / 2 en volumes)]. Après concentration des fractions sous pression réduite, on obtient 1,9 g de (3R, 4R)-1-*tert*-butyloxycarbonyl-3-(1RS,2-dihydroxy-éthyl)-4-[3-(3-fluoro-6-méthoxy-quinolin-4-yl)-3-oxo-propyl]-pipéridine, sous forme d'une huile jaune.

### Spectre de MS (IC) m/z 477, (M+H)+

Le (3R,4R)-1-*tert*-butyloxycarbonyl-4-[3-(3-fluoro-6-méthoxy-quinolin-4-yl)-3-oxo-propyl]-3-vinyl-pipéridine peut être préparé de la manière suivante :

A 3,57 cm³ (25,4 mmol) de diisopropylamine en solution dans 20 cm³ de tétrahydrofurane, on ajoute à une température voisine de -78°C, sous atmosphère d'argon, 15,9 cm³ d'une solution de nBuli 1,6M/hexane. Après retour à une température voisine de 0°C pendant une période de 10 minutes, le milieu réactionnel est refroidi de nouveau à une température voisine de -78°C. On ajoute alors, sous atmosphère d'argon, une solution de 2,97 g (16,8 mmol) de 3-fluoro-6-méthoxy-quinoléine dans 40 cm³ de tétrahydrofurane. Après 4 heures d'agitation à une température voisine de -78°C puis retour à une température voisine de -40°C, on ajoute sous atmosphère d'argon, une solution de 6 g de (17,6 mmol) de (3R, 4R)-1-*tert-*butyloxycarbonyl-4-(2-méthoxycarbonyl-ethyl)-3-vinyl-pipéridine dans 40 cm³ de tétrahydrofurane. Après 10 minutes d'agitation à une température voisine de -40°C puis retour en 10 minutes à une température voisine de -10°C, on ajoute successivement 30 cm³ d'une solution aqueuse saturée avec du chlorure d'ammonium, 30 cm³ d'acétate d'éthyle et 30 cm³ d'eau. La phase organique est décantée, lavée successivement par 2 fois 40 cm³ d'eau et 40 cm³ d'une solution aqueuse saturée en chlorure de sodium, séchée sur sulfate de magnésium anhydre, filtrée et concentrée à sec sous pression réduite (2,7 kPa) pour donner 8 g d'une huile orange qui est purifiée par chromatographie-flash [éluant : dichlorométhane / méthanol / acétonitrile (100 / 0 / 0 puis 99 / 0,5 / 0,5 en volumes)]. Après concentration des fractions sous pression réduite, on obtient 2,47 g de (3R, 4R)-1-*tert-*butyloxycarbonyl-4-[3-(3-fluoro-6-méthoxy-quinolin-4-yl)-3-oxo-propyl]-3-vinyl-pipéridine, sous forme d'une huile jaune.

### Spectre de MS (IC) m/z 443, (M+H)+

Le (3R,4R)-1-tert-butyloxycarbonyl-4-(2-méthoxycarbonyl-éthyl)-3-vinyl-pipéridine peut être préparé de la manière suivante :

A 5 g (17,64 mmol) de (3R,4R)-1-tert-butyloxycarbonyl-4-(2-carboxy-éthyl)-3-vinyl-pipéridine en solution dans 50 cm³ de méthanol, on ajoute à une température voisine de 15°C, sous atmosphère d'argon, 50 cm³ d'une solution de triméthylsilyldiazométhane 2M/hexane. Après 3 heures d'agitation à une température voisine de 22°C, le milieu réactionnel est concentré à sec sous pression réduite (2,7 kPa) pour donner 5,3 g de (3R,4R)-1-*tert*-butyloxycarbonyl-4-(2-méthoxycarbonyl-éthyl)-3-vinyl-pipéridine, sous forme d'une huile orange.

### Spectre de MS (IE) m/z 297 (M+.) m/z 57 (pic de base)

Le (3R,4R)-1-tert-butyloxycarbonyl-4-(2-carboxy-éthyl)-3-vinyl-pipéridine peut être préparé de la manière suivante :

A 2 g (3,7 mmol) de (3R,4R)-1-*tert*-butyloxycarbonyl-4-{3-[*tert*-butyloxycarbonyl-(6-méthoxy-quinolin-4-yl)-amino]-3-oxo-propyl}-3-vinyl-pipéridine en solution dans 56 cm³ de tétrahydrofurane et 18 cm³ d'eau, on ajoute successivement à une température voisine de 20°C, sous atmosphère d'argon, 2,26 cm³ d'une solution aqueuse de peroxyde d'hydrogène à 9,8 mol/L et 0,310 g d'hydroxyde de lithium monohydraté. Après 16 heures d'agitation, on ajoute 50 cm³ d'une solution aqueuse saturée en sulfite de sodium puis on évapore le tétrahydrofurane sous pression réduite (2,7 kPa). La phase aqueuse est filtrée et le résidu est lavé par 3 fois 30 cm³ d'eau. Les filtrats sont réunis, acidifiés à un pH voisin de 3,5 par addition d'acide acétique puis extrait par 3 fois 40 cm³ de dichlorométhane. Les phases organiques sont réunies, séchées sur sulfate de magnésium anhydre, filtrées et concentrées à sec sous pression réduite (2,7 kPa) pour donner 0,9 g de (3R,4R)-1-*tert*-butyloxycarbonyl-4-(2-carboxy-éthyl)-3-vinyl-pipéridine, sous forme d'une huile incolore.

### Spectre de MS (IC) m/z 284, (M+H)+ m/z 301, (M+NH4)+

Le (3R,4R)-1-tert-butyloxycarbonyl-4-{3-[tert-butyloxycarbonyl-(6-méthoxy-quinolin-4-yl)-amino]-3-oxo-propyl}-3-vinyl-pipéridine peut être préparé de la manière suivante :

A 5,8 g (13,21 mmol) de (3R,4R)-1-tert-butyloxycarbonyl-4-[3-(6-méthoxy-quinolin-4-ylamino)-3-oxo-propyl]-3-vinyl-pipéridine en solution dans 150 cm³ de dichlorométhane, on ajoute successivement à une température voisine de 20°C, sous atmosphère d'argon, 1,85 cm³ de triéthylamine , 11,5 g de dicarbonate de di-*tert*-butyle et 1,61 g de 4-(diméthylamino)pyridine. Après 16 heures d'agitation, le milieu réactionnel est lavé successivement par 2 fois 200 cm³ d'eau puis 2 fois 200 cm³ d'une solution aqueuse saturée en chlorure d'ammonium. La phase organique est séchée sur sulfate de magnésium anhydre, filtrée et concentrée à sec sous pression réduite (2,7 kPa) pour donner 8,2 g de (3R,4R)-1-*tert*-butyloxycarbonyl-4-{3-[*tert*-butyloxycarbonyl-(6-méthoxy-quinolin-4-yl)-amino]-3-oxo-propyl}-3-vinyl-pipéridine, sous forme d'une huile orange.

### Spectre de MS (IC) m/z 540, (M+H)+

Le (3R, 4R)-1-*tert*-butyloxycarbonyl-4-[3-(6-méthoxy-quinolin-4-ylamino)-3-oxo-propyl]-3-vinyl-pipéridine peut être préparé de la manière suivante :

A 2,5 g (5,69 mmol) de (3R,4R)-1-*tert*-butyloxycarbonyl-4-[3-[(E,Z)-hydroxyimino]-3-(6-méthoxy-quinolin-4-yl)-propyl]-3-vinyl-pipéridine en solution dans 12,75 cm³ d'acétone, on ajoute successivement à une température voisine de 20°C, sous atmosphère d'argon, 1,73 g de chlorure de p-toluène sulfonyle puis une solution de 0,322 g d'hydroxyde de potassium dans 4,5 cm³ d'eau. Après 30 minutes d'agitation à reflux, le milieu réactionnel est concentré à sec sous pression réduite (2,7 kPa). Le résidu est repris avec 100 cm³ de CH₂Cl₂ puis lavé par 3 fois 50 cm³ d'eau. La phase organique est séchée sur sulfate de magnésium anhydre, filtrée et concentrée à sec sous pression réduite (2,7 kPa) pour donner 3,2 g d'un solide orange qui est purifié par chromatographie-flash [éluant : dichlorométhane / méthanol / acétonitrile (96 / 2 / 2 en volumes)]. Après concentration des fractions sous pression réduite, on obtient 1 g de (3R,4R)-1-tert-butyloxycarbonyl-4-[3-(6-méthoxy-quinolin-4-ylamino)-3-oxo-propyl]-3-vinyl-pipéridine, sous forme d'un solide beige.

### Spectre de MS (IC) m/z 440, (M+H)+

Le (3R,4R)-1-*tert*-butyloxycarbonyl-4-[3-[(E,Z)-hydroxyimino]-3-(6-méthoxy-quinolin-4-yl)-propyl]-3-vinyl-pipéridine peut être préparé de la manière suivante :

A 6,4 g (13,85 mmol) de (3R,4R)-1-*tert*-butyloxycarbonyl-4-[3-(6-méthoxy-quinolin-4-yl)-3-oxo-propyl]-3-vinyl-pipéridine en solution dans 5 cm³ de méthanol et 75 cm³ d'eau, on ajoute successivement à une température voisine de 20°C, 1,2 g de chlorhydrate d'hydroxylamine puis 2,35 g d'acétate de sodium trihydraté. Après 72 heures d'agitation, le milieu réactionnel est extrait avec 100 cm³ puis 2 fois 50 cm³ de dichlorométhane. Les phases organiques sont regroupées, lavées par 100 cm³ d'eau, séchées sur sulfate de magnésium anhydre, filtrées et concentrées à sec sous pression réduite (2,7 kPa) pour donner 5,9 g de (3R,4R)-1-tert-butyloxycarbonyl-4-[3-[(E,Z)-hydroxyimino]-3-(6-méthoxy-quinolin-4-yl)-propyl]-3-vinyl-pipéridine, sous forme d'un solide orange.

### Spectre de MS (IC) m/z 440, (M+H)+

Le (3R,4R)-1-*tert*-butyloxycarbonyl-4-[3-(6-méthoxy-quinolin-4-yl)-3-oxo-propyl]-3-vinyl-pipéridine peut être préparé de la manière suivante :

A 5 g (13,85 mmol) de (3R,4R)-4-[3-(6-méthoxy-quinolin-4-yl)-3-oxo-propyl]-3-vinyl-pipéridine en solution dans 100 cm³ de dichlorométhane, on ajoute successivement à une température voisine de 20°C, sous atmosphère d'argon, 4,28 cm³ de triéthylamine puis une solution de 3,43 g de dicarbonate de di-*tert*-butyle dans 50 cm³ de dichlorométhane. Après 16 heures d'agitation, le milieu réactionnel est lavé par 3 fois 150 cm³ d'eau. La phase organique est séchée sur sulfate de magnésium anhydre, filtrée et concentrée à sec sous pression réduite (2,7 kPa) pour donner 6,4 g de (3R,4R)-1-tert-butyloxycarbonyl-4-[3-(6-méthoxy-quinolin-4-yl)-3-oxo-propyl]-3-vinyl-pipéridine, sous forme d'une huile orange.

### Spectre de MS (IC) m/z 425, (M+H)+

La (3R,4R)-4-[3-(6-méthoxy-quinolin-4-yl)-3-oxo-propyl]-3-vinyl-pipéridine peut être préparé par application de la méthode décrite dans la demande de brevet FR 2354771.

### Exemple 2

### Acide (3R,4R)- 4- [3-oxo-3- (3-fluoro-6-méthoxyquinolin-4-yl) - propyl]- 1-[2-(2,5-difluoro-phénylsulfanyl)-éthyl]-pipéridine-3-carboxylique

Un mélange de 0,1 g de (3R,4R)-4-[3-oxo-3-(3-fluoro-6-méthoxyquinolin-4-yl)-propyl]-1-[2-(2,5-difluoro-phénylsulfanyl)-éthyl]-pipéridine-3-carboxylate de méthyle dans 2 cm³ d'acide chlorhydrique 5N , est porté à une température voisine de 80°C sous agitation et atmosphère inerte pendant 5 heures. Après refroidissement aux environs de 20°C, le milieu réactionnel est neutralisé par 1,8 cm³ de soude 5N jusqu'au PH 6 et ensuite extrait par 20 cm³ de dichlorométhane. La phase organique est séchée sur du sulfate de magnésium, filtrée puis évaporée à sec sous pression réduite (2 kPa) à une température voisine de 30°C. Le résidu obtenu est purifié par chromatographie sur une colonne de gel de silice (granulométrie 70-200 µm ; diamètre 2 cm), en éluant par un mélange de dichlorométhane-méthanol-ammoniaque (28%) (40/5/0,5 en volumes) et en recueillant des fractions de 10 cm³. Les fractions contenant le produit attendu sont réunies puis évaporées selon les conditions décrites précédemment. On obtient 0,072 g d'acide (3R,4R)-4-[3-oxo-3-(3-fluoro-6-méthoxyquinolin-4-yl)-propyl]-1-[2-(2,5-difluoro-phénylsulfanyl)-éthyl]-pipéridine-3-carboxylique, sous forme d'un solide de couleur beige.

Spectre de R.M.N. ¹H (300 MHz, (CD₃)₂SO d6, δ en ppm) : de 1,55 à 1,90 (mt : 5H) ; 2,29 (mt : 1H) ; 2,44 (d large, J = 12 Hz : 1H) ; 2,61 (mt : 1H) ; 2,68 (t, J = 7 Hz : 2H) ; 2, 79 (mt : 1H) ; 2,97 (mt : 1H) ; 3,12 (mt : 2H) ; 3,20 (t large, J = 6 Hz : 2H) ; 3,91 (s : 3H) ; de 7,00 à 7,15 (mt : 1H) ; 7,09 (d, J = 3 Hz : 1H) ; de 7,20 à 7,40 (mt : 2H) ; 7,47 (dd, J = 9 et 3 Hz : 1H) ; 8,05 (d, J = 9 Hz : 1H) ; 8,89 (d, J = 0,5 Hz . 1H) ; de 12,80 à 13,20 (mf étalé : 1H).

### (3R,4R)-4-[3-oxo-3-(3-fluoro-6-méthoxyquinolin-4-yl)-propyl]- 1-[2-(2,5-difluoro-phénylsulfanyl)-éthyl]-pipéridine-3-carboxylate de méthyle

Un mélange de 0,57 g de (3R,4R)-4-[3-oxo-3-(3-fluoro-6-méthoxyquinolin-4-yl)-propyl]-pipéridine-3-carboxylate de méthyle obtenu comme décrit à l'exemple 1, 0,462 g de 2-(2-bromo-éthylsulfanyl)-1,4-difluoro-benzène, 0,253 g d'iodure de potassium et 1,05 g de carbonate de potassium dans 25 cm³ d'acétonitrile est chauffé sous agitation et sous atmosphère inerte pendant 20 heures à une température voisine de 75°C. Après refroidissement à une température voisine de 20°C, le milieu réactionnel est filtré et l'insoluble est lavé par 2 fois 10 cm³ d'acétonitrile. Le filtrat est évaporé sous pression réduite (2 kPa) à une température voisine de 40°C. Le résidu d'évaporation est repris par 50 cm³ d'eau distillée et 100 cm³ d'acétate d'éthyle. La phase organique est lavée par 3 fois 30 cm³ d'eau distillée et 2 fois 50 cm³ d'une solution aqueuse saturée en chlorure de sodium, séchée sur sulfate de magnésium et évaporée selon les conditions décrites précédemment. L'huile obtenue est purifiée par chromatographie , sur une colonne de gel de silice (granulométrie 70-200 µm ; diamètre 2.5 cm), en éluant par un mélange de dichlorométhane-méthanol (90/10 en volumes) et en recueillant des fractions de 10 cm³. Les fractions contenant le produit attendu sont réunies puis évaporées sous pression réduite (2 kPa) à une température voisine de 40°C. On obtient 0.4 g de (3R,4R)-4-[3-oxo-3-(3-fluoro-6-méthoxyquinolin-4-yl)-propyl]- 1-[2-(2,5-difluoro-phénylsulfanyl)-éthyl]-pipéridine-3-carboxylate de méthyle, sous forme d'une huile visqueuse de couleur orange.

Spectre de R.M.N. ¹H (300 MHz, (CD₃)₂SO d6, δ en ppm) : 1,56 (mt : 1H) ; de 1,65 à 2,00 (mt : 4H) ; 2,24 (mt : 1H) ; 2,40 (d large, J = 12 Hz : 1H) ; de 2,50 à 2,70 (mt : 3H) ; 2,72 (mt : 1H) ; 2,85 (mt : 1H) ; 3,04 (mt : 2H) ; 3,12 (t, J = 7 Hz : 2H) ; 3,57 (s : 3H) ; 3, 90 (s : 3H) ; de 7,00 à 7,10 (mt : 1H) ; 7,09 (d, J = 3 Hz : 1H) ; de 7,20 à 7,35 (mt : 2H) ; 7,48 (dd, J = 9 et 3 Hz : 1H) ; 8,05 (d, J = 9 Hz : 1H) ; 8,89 (s : 1H).

### Exemple 3

### Acide (3RS,4RS)- 4-[3-oxo-3-(3-fluoro-6-méthoxyquinolin-4-yl)-propyl]- 1-[2-(2,5-difluoro-phénylsulfanyl)-éthyl]-pipéricline-3-acétique

Un mélange de 0,3 g de (3RS,4RS)-4-[3-oxo-3-(3-fluoro-6-méthoxyquinolin-4-yl)-propyl]-1-[2-(2,5-difluoro-phénylsulfanyl)-éthyl]-pipéridine-3-acétate de méthyle dans 1,34 cm³ d'une solution aqueuse de soude 1N et 5 cm³ de dioxane, est porté à une température voisine de 60°C sous agitation et atmosphère inerte pendant 1 heure. Après refroidissement aux environs de 20°C, le milieu réactionnel est évaporé à sec sous pression réduite (2 kPa) à une température voisine de 50°C. Le résidu d'évaporation obtenu est repris par 20 cm³ d'eau et 20 cm³ d'éther diéthylique, la phase aqueuse est décantée et neutralisée par 1,3 cm³ d'une solution aqueuse d'acide chlorhydrique 1N et est ensuite extraite par 70 cm³ d'acétate d'éthyle. La phase organique est séchée sur du sulfate de magnésium, filtrée puis évaporée selon les mêmes conditions précédentes. On obtient 0,23 g d'acide (3RS,4RS)-4-[3-oxo-3-(3-fluoro-6-méthoxyquinolin-4-yl)-propyl]- 1-[2-(2,5-difluoro-phénylsulfanyl)-éthyl]-pipéridine-3-acétique, sous forme d'un solide de couleur beige.

Spectre de R.M.N. ¹H (300 MHz, (CD₃)₂SO d6, δ en ppm) : 1,32 (t très large, J = 13,5 Hz : 1H) ; 1,49 (d large : J = 13,5 Hz : 1H) ; de 1,50 à 1,75 (mt : 3H) ; de 1,90 à 2,20 (mt : 3H) ; 2,20 (d mt, J = 16,5 Hz : 1H) ; 2,47 (dd, J = 16,5 et 9 Hz : 1H) ; de 2,50 à 2,70 (mt : 2H) ; 2,78 (mt : 2H) ; 3,06 (t, J = 7,5 Hz : 2H) ; 3,12 (t, J = 7 Hz : 2H) ; 3,90 (s : 3H) ; de 7,00 à 7,15 (mt : 1H) ; 7,10 (d, J = 3 Hz : 1H) ; de 7,20 à 7,35 (mt : 2H) ; 7,47 (dd, J = 9 et 3 Hz : 1H) ; 8,04 (d, J = 9 Hz : 1H) ; 8,88 (s : 1H) .

### (3RS,4RS)-4-[3-oxo-3-(3-fluoro-6-méthoxyquinolin-4-yl)-propyl]-1-[2-(2,5-difluoro-phénylsulfanyl)-éthyl]-pipéridine-3-acétate de méthyle

A une solution de 1,32 cm³ de dichlorure d'oxalyle dans 30 cm³ de dichlorométhane refroidie à -70°C, sous agitation et atmosphère inerte, on coule 1,88 cm³ de diméthylsulfoxyde dans 6 cm³ de dichlorométhane en 10 minutes. Au bout de 10 minutes, on coule une solution de 1,7 g de (3RS,4RS)-4-[3-(R,S)-hydroxy-3-(3-fluoro-6-méthoxyquinolin-4-yl)-propyl]-1-[2-(2,5-difluoro-phénylsulfanyl)-éthyl]-pipéridine-3-acétate de méthyle dans 15 cm³ de dichlorométhane en 10 minutes. Au bout de 15 minutes, on ajoute goutte à goutte 8,4 cm³ de triéthylamine dans 10 cm³ de dichlorométhane en 15 minutes et le milieu réactionnel est agité pendant 45 minutes au voisinage de -70°C puis 20 heures au voisinage de 20°C. On coule sur la masse réactionnelle 50 cm³ d'eau distillée, la phase organique est décantée, lavée par 50 cm³ d'une solution aqueuse d'hydrogénocarbonate de sodium saturée, par 2 fois 30 cm³ d'eau distillée et 30 cm³ d'une solution aqueuse de chlorure de sodium saturée. L'extrait organique est séché sur du sulfate de magnésium, filtré et évaporé sous pression réduite (2 kPa) à une température voisine de 40°C. Le résidu obtenu est purifié par chromatographie sous une pression d'argon de 50 kPa, sur une colonne de gel de silice (granulométrie 40-60 µm ; diamètre 3 cm), en éluant par un mélange de cyclohexane-acétate d'éthyle (60/40 en volumes) et en recueillant des fractions de 10 cm³. Les fractions contenant le produit attendu sont réunies puis évaporées selon les conditions décrites précédemment. On obtient 1,37 g de (3RS,4RS)-4-[3-oxo-3-(3-fluoro-6-méthoxyquinolin-4-yl)-propyl]-1-[2-(2,5-difluoro-phénylsulfanyl)-éthyl]-pipéridine-3-acétate de méthyle.
Spectre infra-rouge : (CCl₄) 2930; 1737; 1701; 1621; 1506; 1484; 1468; 1232; 1189; 1166; 1028; 905 et 834 cm⁻¹

### (3RS,4RS)-4-[3-(R,S)-hydroxy-3-(3-fluoro-6-méthoxyquinolin-4-yl)-propyl]- 1-[2-(2,5-difluoro-phénylsulfanyl)-éthyl]-pipéridine-3-acétate de méthyle

Un mélange de 6,5 g de dichlorhydrate du (3RS,4RS)-4-[3-(R,S)-hydroxy-3-(3-fluoro-6-méthoxyquinolin-4-yl)-propyl]-pipéridine-3-acétate de méthyle, 3,9.g de 2-(2-bromo-éthylsulfanyl)-1,4-difluoro-benzène dissout dans 10 cm³ de diméthylformamide, 2,32 g d'iodure de potassium, 5,8 g de carbonate de potassium et 3,93 cm³ de triétylamine dans 200 cm³ d'acétonitrile est chauffé sous agitation et sous atmosphère inerte pendant 22 heures à une température voisine de 70°C . Après refroidissement à une température voisine de 20°C, le milieu réactiofinel est filtré et l'insoluble est lavé par 2 fois 30 cm³ d'acétonitrile. Le filtrat est évaporé sous pression réduite (2 kPa) à une température voisine de 40°C. Le résidu d'évaporation est repris par 100 cm³ d'eau distillée et 150 cm³ d'acétate d'éthyle. La phase organique est lavée par 3 fois 100 cm³ d'eau distillée et 2 fois 100 cm³ d'une solution aqueuse saturée en chlorure de sodium, séchée sur sulfate de magnésium et évaporée selon les conditions décrites précédemment. L'huile obtenue est purifiée par chromatographie sous pression d'argon de 50 kPa , sur une colonne de gel de silice (granulométrie 40-60 µm ; diamètre 4 cm), en éluant par un mélange de cyclohexane-acétate d'éthyle (50/50 en volumes) et en recueillant des fractions de 60 cm³. Les fractions contenant le produit attendu sont réunies puis évaporées sous pression réduite (2 kPa) à une température voisine de 40°C. On obtient 1,7 g de (3RS,4RS)-4-[3-(R,S)-hydroxy-3-(3-fluoro-6-méthoxyquinolin-4-yl)-propyl]- 1-[2-(2,5-difluoro-phénylsulfanyl)-éthyl]-pipéridine-3-acétate de méthyle, sous forme d'une huile visqueuse de couleur orange.

Spectre de R.M.N. ¹H (300 MHz, (CD₃)₂SO d6, δ en ppm) . Nous observons le mélange de deux diastéréoisomères dans les proportions 50/50 : de 0,90 à 2,60 (mt : 14H) ; de 2,60 à 2,80 (mt : 2H) ; 3,08 (t large, J = 7 Hz : 2H) ; 3,47 et 3,55 (2 s : 3H en totalité) ; 3,89 (s : 3H) ; 5,33 (t très large, J = 7 Hz : 1H) ; 5,83 (s large : 1H) ; 7,05 (mt : 1H) ; de 7,15 à 7,35 (mt : 2H) ; 7,38 (d mt, J = 9 Hz : 1H) ; de 7,90 à 8,00 (mt : 2H) ; 8,68 (s large : 1H) .

### Dichlorhydrate du (3RS,4RS) et (3SR,4RS)-4-[3-(R,S)-hydroxy-3-(3-fluoro-6-méthoxyquinolin-4-yl)-propyl]-pipéridine-3-acétate de méthyle.

Une solution de 940 mg d'acide (3RS, 4RS) et (3SR, 4RS)-4-[3-(R,S)-hydroxy-3-(3-fluoro-6-méthoxyquinolin-4-yl)propyl]-1-(tert-butyloxycarbonyl)-pipéridine-3-acétique dans 20 cm³ de méthanol est refroidie à une température voisine de -25°C, sous agitation et sous atmosphère inerte. A cette solution est ajoutée en 5 minutes 0,43 cm³ de chlorure de thionyle. Le mélange est ramené à une température voisine de 20°C, tandis que l'agitation est poursuivie pendant encore 1 heure 30 minutes. On concentre le mélange réactionnel sous pression réduite (5 kPa) à une température voisine de 40°C puis on ajoute 30 cm³ de méthanol. On renouvelle cette série d'opérations 3 fois. On obtient 920 mg de dichlorhydrate de (3RS, 4RS) et (3SR, 4RS)-4-[3-(R,S)-hydroxy-3-(3-fluoro-6-méthoxyquinolin-4-yl)propyl]-pipéridine-3-acétate de méthyle, sous forme d'une mousse jaune.

Spectre infra rouge (KBr) : 3249; 1949; 2503; 2020; 1731; 1622; 1604; 1555; 1497; 1457; 1420; 1308; 1242; 1200; 1175; 1080; 1014; 872; 832 et 795 cm⁻¹

Acide (3RS, 4RS) et (3SR, 4RS)-4-[3-(R,S)-hydroxy-3-(3-fluoro-6-méthoxyquinolin-4-yl)propyl]-1-(tert-butyloxy-carbonyl)-pipéridine-3-acétique.

Une solution de 1,16g de (3RS,4RS) et (3SR,4RS)-4-[3-(3-fluoro-6-méthoxyquinolin-4-yl)-3-propyl]-1-(tert-butyloxycarbonyl)-pipéridine-3-acétate de méthyle, 100 cm³ de diméthylsulfoxyde anhydre et 25 cm³ de tert-butanol anhydre est agitée sous atmosphère inerte exempte d'eau à 20°C. On purge cette solution incolore avec de l'oxygène pur jusqu'à saturation du mélange réactionnel. On ajoute alors une solution contenant 685 mg de tert-butoxyde de potassium dans 8 cm³ de tert-butanol anhydre. L'oxygène est de nouveau introduit par barbotage pendant encore 3 heures et 30 minutes sous forte agitation. La solution jaune obtenue est purgée à l'azote puis refroidie à 0°C. On ajoute ensuite 0,5 cm³ d'acide acétique pur dans 20 cm³ d'eau puis 200 cm³ d'éther. La phase organique est décantée, lavée par 7 fois 20 cm³ d'eau et par 3 fois 20 cm³ d'une solution aqueuse saturée de chlorure de sodium, séchée sur sulfate de magnésium, filtrée et concentrée sous pression réduite (5 kPa) à une température voisine de 40°C. On obtient une gomme que l'on reprend dans 20 cm³ d'éther. On concentre de nouveau dans les mêmes conditions que précédemment. On obtient 945 mg d'acide (3RS,4RS) et (3SR,4RS)-4-[3-(R,S)-hydroxy-3-(3-fluoro-6-méthoxyquinolin-4-yl)propyl]-1-(tert-butyloxycarbonyl)-pipéridine-3-acétique, sous forme d'une mousse blanche.

Spectre infra rouge (KBr) : 2973; 2932; 2864; 1693; 1668; 1623; 1510; 1468; 1429; 1366; 1232; 1166; 1030 et 831 cm⁻¹

Spectre infra rouge (CH₂Cl₂) : 3600; 2982; 2939; 2867; 1710; 1682; 1623; 1509; 1468; 1429; 1367; 1231; 1162; 1030; 909; 896 et 834 cm⁻¹

(3RS, 4RS) et (3SR, 4RS)-4-[3-(3-fluoro-6-méthoxyquinolin-4-yl)-3-propyl]-1-(tert-butyloxycarbonyl )-pipéridine-3-acétate de méthyle.

Une solution de 1,85 g de (3RS,4RS) et (3SR,4RS)-4-[3-(3-fluoro-6-méthoxyquinolin-4-yl)propyl]-pipéridine-3-acétate de méthyle, 0,7 cm³ de triéthylamine et 40 cm³ de dichlorométhane est refroidie, à une température voisine de 0°C, sous agitation et sous atmosphère d'argon. A cette solution incolore est ajoutée en 20 minutes une solution de 1,16 g de di-tert-butyldicarbonate dissous dans 40 cm³ de dichlorométhane. Le mélange est ramené à une température voisine de 20°C, tandis que l'agitation est poursuivie pendant encore 10 heures. On ajoute alors au mélange réactionnel 200 cm³ d'eau. La phase organique est décantée, lavée par 100 cm³ d'une solution aqueuse saturée de chlorure de sodium, séchée sur sulfate de magnésium, filtrée, puis concentrée sous pression réduite (5 kPa) à une température voisine de 40°C. On obtient une huile que l'on purifie par chromatographie, sous une pression d'azote de 50 kPa, sur une colonne de gel de silice (granulométrie 20-45µ ; diamètre 2 cm; hauteur 20 cm), en éluant par un mélange de cyclohexane-acétate d'éthyle (70/30 en volumes) et en recueillant des fractions de 40 cm³. Les fractions 8 à 12 sont réunies, puis concentrées comme précédemment. On obtient 2,16 g de (3RS,4RS) et (3SR,4RS)-4-[3-(3-fluoro-6-méthoxyquinolin-4-yl)-3-propyl]-1-(tert-butyloxycarbonyl)-pipéridine-3-acétate de méthyle, sous forme d'une huile incolore.

Spectre infra rouge (CCl₄ 3006; 1740; 1695; 1622; 1507; 1468; 1428; 1366; 1231; 1166; 1034; 909 et 832 cm⁻¹

(3RS, 4RS) et (3SR, 4RS)-4-[3-(3-Fluoro-6-méthoxyquinolin-4-yl)propyl]-pipéridine-3-acétate de méthyle.

Dans un autoclave, on introduit 2,65 g de (4RS)-1-benzyloxycarbonyl-4-[3-(3-fluoro-6-méthoxyquinolin-4-yl) propyl]-pipéridine-3-ylidène acétate de méthyle, isomère Z, 45 cm³ d'éthanol absolu et 265 mg de palladium sur charbon à 10 %. Le mélange réactionnel est agité sous 5 bars d'hydrogène à 22°C pendant 24 heures, puis, filtré sur supercel, rincé avec 5 fois 20 cm³ d'éthanol absolu. Les filtrats réunis sont concentrés sous pression réduite (5 kPa) à une température voisine de 40°C. On obtient 1,85 g de (3RS, 4RS) et (3SR, 4RS)-4-[3-(3-fluoro-6-méthoxyquinolin-4-yl)propyl]-pipéridine-3-acétate de méthyle, sous forme d'une huile incolore.

Spectre de R.M.N. ¹H (300 MHz, (CD₃)₂SO d6, δ en ppm). de 1,10 à 1,80 (mt : 7H) ; de 1,90 à 2,30 (mt : 2H) ; de 2,35 à 2,60 (mt : 3H) ; de 2,65 à 2,95 (mt : 2H) ; 3,06 (mt : 2H) ; 3,55 et 3,56 (2s : 3H en totalité) ; 3,95 et 3,96 (2s : 3H en totalité) ; de 7,30 à 7,45 (mt : 2H) ; 7,96 (d, J = 9 Hz : 1H) ; 8,70 (s large : 1H).

(4RS)-1-benzyloxycarbonyl-4-[3-(3-Fluoro-6-méthoxyquinolin-4-yl) propyl]-pipéridine-3-ylidène acétate de méthyle, isomère Z.

Une solution de 5,8 g de (4RS)-4-allyl-1-benzyloxycarbonyl pipéridin-3-ylidène acétate de méthyle (isomère Z) dans 15 cm³ de tétrahydrofurane est ajoutée lentement, à une température voisine de 0°C, sous agitation et sous atmosphère inerte, à 45 cm³ d'une solution 0,5 M de 9-borabicyclo[3,3,1]nonane dans le tétrahydrofurane. Le mélange est ensuite ramené à une température voisine de 20°C, tandis que l'agitation est poursuivie pendant encore 4 heures. 5,5 g de 4-iodo-3-fluoro-6-méthoxy quinoléine en solution dans 100cm³ de tétrahydrofurane sont ajoutés, puis 11,2 g de phosphate de potassium tribasique, et enfin 386 mg de chlorure de palladium diphénylphosphinoferrocène. Le mélange réactionnel est chauffé pendant 2 heures au reflux puis agité 48 heures à température ambiante. La suspension obtenue est filtrée. Le filtrat est concentré puis repris dans 200 cm³ d'acétate d'éthyle. La solution obtenue est lavée par 2 fois 200 cm³ d'eau puis par 2 fois 200 cm³ d'une solution aqueuse saturée de chlorure de sodium, séchée sur sulfate de magnésium, filtrée, puis concentrée sous pression réduite (5 kPa) à une température voisine de 40°C. On obtient 15 g d'une huile que l'on purifie par chromatographie, sous une pression d'azote de 50 kPa, sur une colonne de gel de silice (granulométrie 20-45µ ; diamètre 6 cm ; hauteur 38 cm), en éluant par un mélange de cyclohexane-acétate d'éthyle (85/15 en volumes en faisant un gradient jusqu'à 70/30 en volumes) et en recueillant des fractions de 200 cm³. Les fractions 31 à 34 sont réunies, puis concentrées. On obtient 4,7 g de (4RS)-1-benzyloxycarbonyl-4-[3-(3-fluoro-6-méthoxyquinolin-4-yl) propyl]-pipéridine-3-ylidène acétate de méthyle (isomère Z), sous forme d'une huile incolore.

Spectre infra rouge (CCl₄): 3091; 3068; 3034; 1705; 1655; 1622; 1507; 1468; 1434; 1361; 1263; 1231; 1207; 1173; 1141; 1034; 909; 832 et 696 cm⁻¹

(4RS)-4-allyl-1-benzyloxycarbonyl pipéridin-3-ylidène acétate de méthyle, isomère Z.

Une solution contenant 16,3 g de (4RS)-4-allyl-1-benzyloxy-carbonyl pipéridin-3-one dans 200 cm ³ de toluène est agitée au reflux avec du (triphénylphosphoranylidène) acétate de méthyle, sous atmosphère inerte, pendant 16 heures. Après refroidissement à environ 20°C, le mélange réactionnel est concentré sous pression réduite (5 kPa) à une température voisine de 40°C, le résidu obtenu, solubilisé dans 50 cm³ de dichlorométhane à chaud, est purifié par chromatographie, sous une pression d'azote de 50 kPa, sur une colonne de gel de silice (granulométrie 20-45µ ; diamètre 10 cm; hauteur 45 cm), en éluant par un mélange de cyclohexane-acétate d'éthyle (80/20 en volumes) et en recueillant des fractions de 250 cm³. Les fractions 13 à 15 sont réunies, puis concentrées comme ci-dessus. On obtient 5,8 g de (4RS)-4-allyl-1-benzyloxy-carbonyl pipéridin-3-ylidène acétate de méthyle (isomère Z), sous forme d'une huile incolore.

Spectre infra rouge (CCl₄) : 3068; 3034; 2949; 2853; 1722; 1705; 1655; 1643; 1434; 1260; 1200; 1174; 1144; 993; 918 et 696 cm⁻¹

La (4RS)-4-allyl-1-benzyloxycarbonyl-piperidin-3-one peut être préparée selon Takeuchi Y et coll. décrit dans Synthesis 1999, 10, 1814.

### Exemple 4

### Acide (3R,4R)- 4-[3-oxo-3-(6-méthoxyquinolin-4-yl)-propyl]- 1-[2-(2-thiénylsulfanyl)-éthyl]-pipéridine-3-carboxylique

Un mélange de 0,43 g de (3R,4R)-4-[3-oxo-3-(6-méthoxyquinolin-4-yl)-propyl]-1-[2-(2-thiénylsulfanyl)-éthyl]-pipéridine-3-carboxylate de méthyle dans 5 cm³ d'acide chlorhydrique 5N , est porté à une température voisine de 80°C sous agitation et atmosphère inerte pendant 5 heures. Après refroidissement aux environs de 20°C, le milieu réactionnel est neutralisé par 4,7 cm³ de soude 5N jusqu'au PH 6 et ensuite extrait par 30 cm³ de dichlorométhane. La phase organique est séchée sur du sulfate de magnésium, filtrée puis évaporée à sec sous pression réduite (2 kPa) à une température voisine de 30°C. Le résidu obtenu est purifié par chromatographie sur une colonne de gel de silice (granulométrie 70-200 µm ; diamètre 2 cm), en éluant par un mélange de chloroforme-méthanol-ammoniaque (28%) (12/3/0,5 en volumes) et en recueillant des fractions de 10 cm³. Les fractions contenant le produit attendu sont réunies puis évaporées selon les conditions décrites précédemment. On obtient 0,4 g d'acide (3R,4R)-4-[3-oxo-3-(6-méthoxyquinolin-4-yl)-propyl]- 1-[2-(2-thiénylsulfanyl)-éthyl]-pipéridine-3-carboxylique, sous forme d'un huile de couleur jaune.

Spectre de R.M.N. ¹H (300 MHz, (CD₃)₂SO d6, δ en ppm) : de 1,55 à 1,85 (mt : 5H) ; 2,35 (mt : 1H) ; de 2,40 à 2,50 (mt : 1H) ; de 2,60 à 2,75 (mt : 3H) ; 2,84 (mt : 1H) ; de 2,90 à 3,10 (mt : 3H) ; 3,19 (mt : 2H) ; 3,89 (s : 3H) ; 7,06 (dd, J = 6 et 3,5 Hz : 1H) ; 7,22 (d large, J = 3,5 Hz : 1H) ; 7,48 (dd, J = 9 et 3 Hz : 1H) ; 7,62 (dd, J = 6 et 1 Hz : 1H) ; 7,73 (d, J = 3 Hz : 1H) ; 7,91 (d, J = 5 Hz : 1H) ; 8,02 ( d, J = 9 Hz : 1H) ; 8,88 (d, J = 5 Hz : 1H) .

### (3R,4R)-4-[3-oxo-3-(6-méthoxyquinolin-4-yl)-propyl]- 1-[2-(2-thiénylsulfanyl)-éthyl]-pipéridine-3-carboxylate de méthyle

Un mélange de 0,95 g de (3R,4R)-4-[3-oxo-3-(6-méthoxy-quinolin-4-yl)-propyl]-pipéridine-3-carboxylate de méthyle, 0,72 g de 2-(2-bromo-éthylsulfanyl)-2-thiophène , 0,44 g d'iodure de potassium et 1,9 g de carbonate de potassium dans 40 cm³ d'acétonitrile est chauffé sous agitation et sous atmosphère inerte pendant 17 heures à une température voisine de 70°C . Après refroidissement à une température voisine de 20°C, le milieu réactionnel est filtré et l'insoluble est lavé par 2 fois 15 cm³ d'acétonitrile. Le filtrat est évaporé sous pression réduite (2 kPa) à une température voisine de 40°C. Le résidu d'évaporation est repris par 50 cm³ d'eau distillée et 100 cm³ d'acétate d'éthyle. La phase organique est lavée par 3 fois 30 cm³ d'eau distillée et 2 fois 50 cm³ d'une solution aqueuse saturée en chlorure de sodium, séchée sur sulfate de magnésium et évaporée selon les conditions décrites précédemment. L'huile obtenue est purifiée par chromatographie , sur une colonne de gel de silice (granulométrie 70-200 µm ; diamètre 2.5 cm), en éluant par un mélange de acétate d'éthyle-éther de pétrole (80/20 en volumes) et en recueillant des fractions de 10 cm³. Les fractions contenant le produit attendu sont réunies puis évaporées sous pression réduite (2 kPa) à une température voisine de 40°C. On obtient 0.43 g de (3R,4R)-4-[3-oxo-3-(6-méthoxyquinolin-4-yl)-propyl]- 1-[2-(2-thiénylsulfanyl)-éthyl]-pipéridine-3-carboxylate de méthyle, sous forme d'une huile visqueuse de couleur brune.

Spectre de R.M.N. ¹H (300 MHz, (CD₃)₂SO d6, δ en ppm) : 1,56 (mt : 1H) ; de 1,60 à 1,95 (mt : 4H) ; 2,21 (mt : 1H) ; 2,36 (d large, J = 12 Hz : 1H) ; de 2,40 à 2,60 (mt : 3H) ; de 2,70 à 2, 85 (mt : 2H) ; 2,91 (t, J = 7 Hz : 2H) ; de 3,00 à 3,30 (mt : 2H) ; 3,57 (s : 3H) ; 3,89 (s : 3H) ; 7,05 (dd, J = 6 et 3,5 Hz : 1H) ; 7,18 (dd, J = 3,5 et 1 Hz : 1H) ; 7,49 (dd, J = 9 et 3 Hz : 1H) ; 7,61 (dd, J = 6 et 1 Hz : 1H) ; 7,73 (d, J = 3 Hz : 1H) ; 7,92 (d, J = 5 Hz : 1H) ; 8,03 (d, J = 9 Hz : 1H) ; 8,90 (d, J = 5 Hz : 1H) .

Le (3R,4R)-4-[3-oxo-3-(6-méthoxyquinolin-4-yl)-propyl]-pipéridine-3-carboxylate de méthyle peut être obtenu par application de la méthode décrite dans la demande de brevet FR 99 11679.

### Exemple 5

### Acide (3R,4R)-4-[3-oxo-3-(6-méthoxyquinolin-4-yl)propyl]-1-[3-(2,3,5-trifluorophényl)prop-2-ynyl]pipéridine-3-carboxylique

Une solution de 1 g de (3R,4R)-4-[3-oxo-3-(6-méthoxyquinolin-4-yl) propyl]-1-[3-(2,3,5-trifluorophényl)prop-2-ynyl]pipéridine-3-carboxylate de méthyle dans 10 cm³ d'une solution aqueuse d'acide chlorhydrique 5N est agitée pendant 4 heures à une température voisine de 80°C. Après refroidissement à une température voisine de 20°C, on ajoute 10 cm³ d'eau, puis 10 cm³ de chloroforme, puis 1,9 g d'hydrogénocarbonate de sodium en poudre. Le mélange est extrait par 3 fois 10 cm³ de chloroforme. Les phases organiques sont réunies, séchées sur sulfate de sodium, filtrées puis concentrées sous pression réduite (5 kPa) à une température voisine de 40°C. Le résidu obtenu est purifié par chromatographie sous une pression de 50 kPa d'azote, sur une colonne de gel de silice (granulométrie 20-45µ ; diamètre 2,5 cm ; 30 g), en éluant par un mélange de dichlorométhane-méthanol (96/4 en volumes) et en recueillant d'abord une fraction de 150 cm³, puis des fractions de 10 cm³. Les fractions 5 à 22 sont réunies, puis concentrées comme précédemment. On obtient 0,583 g d'acide (3R,4R)-4-[3-oxo-3-(6-méthoxyquinolin-4-yl)propyl]-1-[3-(2,3,5-trifluoro-phényl)prop-2-ynyl]pipéridine-3-carboxylique, sous forme d'un solide de couleur verte fondant vers 70°C.

Spectre de R.M.N. ¹H (300 MHz, (CD₃)₂SO d6, δ en ppm) : de 1,60 à 1,95 (mt : 5H) ; 2,44 (mt : 1H) ; 2,58 (d large, J = 11 Hz : 1H) ; 2,73 (mt : 2H) ; 2,92 (mt : 1H) ; 3,19 (mt : 2H) ; 3,67 (s : 2H) ; 3,88 (s : 3H) ; 7,32 (mt : 1H) ; 7,48 (dd, J = 9 et 2,5 Hz : 1H) ; 7,63 (mt : 1H) ; 7,72 (d, J = 2,5 Hz : 1H) ; 7,92 (d, J = 4, 5 Hz : 1H) ; 8,02 (d, J = 9 Hz : 1H) ; 8,89 (d, J = 4,5 Hz : 1H) ; de 12,30 à 12,80 (mf étalé : 1H).

Pouvoir rotatoire : α_{D}²⁰=+27,9° +/-0,8, dans le méthanol à 0,5 %.

### (3R,4R)-4-[3-oxo-3-(6-méthoxyquinolin-4-yl)propyl]-1-[3-(2,3,5-trifluorophényl)prop-2-ynyl]pipéridine-3-carboxylate de méthyle

Un mélange de 17,28 g de (3R,4R)-4-[3-oxo-3-(6-méthoxyquinolin-4-yl) propyl]-1-(prop-2-ynyl)pipéridine-3-carboxylate de méthyle dans 173 cm³ de triéthylamine est agité pendant 5 minutes sous atmosphère inerte à une température voisine de 20°C. On ajoute 4,05 g de tétrakis triphénylphosphine palladium, 0,834 g d'iodure cuivreux et 7,9 g de 1-bromo-2,3,5-trifluorobenzène. Le mélange est agité pendant 2 heures à une température voisine de 80°C. Après refroidissement à environ 20°C, le mélange réactionnel est additionné de 150 cm³ d'acétate d'éthyle et 150 cm³ d'eau, puis décanté. La phase aqueuse est extraite par 3 fois 150 cm³ d'acétate d'éthyle. Les phases organiques sont réunies, lavées par 5 fois 150 cm³ d'eau, séchées sur sulfate de sodium, filtrées, concentrées sous pression réduite (5 kPa) à une température voisine de 40°C. Le résidu obtenu est purifié par chromatographie sous une pression d'azote de 50 kPa, sur une colonne de gel de silice (granulométrie 20-45µ ; diamètre 7 cm ; 600 g), en éluant par de l'acétate d'éthyle pur et en recueillant d'abord une fraction de 2,5 l , puis des fractions de 250 cm³. Les fractions 2 à 29 sont réunies puis concentrées comme ci-dessus. On obtient 18,4 g de (3R,4R)-4-[3-oxo-3-(6-méthoxyquinolin-4-yl)propyl]-1-[3-(2,3,5-trifluoro-phényl)prop-2-ynyl]pipéridine-3-carboxylate de méthyle, sous forme d'une huile jaune.

Spectre de R.M.N. ¹H (400 MHz, (CD₃)₂SO d6, δ en ppm) : de 1,55 à 1,95 (mt : 5H) ; 2,39 (mt : 1H) ; 2,58 (d large, J = 10 Hz : 1H) ; 2,68 (mt : 1H) ; 2,82 (mt : 1H) ; 2,91 (mt : 1H) ; 3,09 (mt : 1H) ; 3,23 (mt :1H) ; 3,58 (s : 3H) ; 3,61 (s : 2H) ; 3,88 (s : 3H) ; 7,31 (mt : 1H) ; 7,49 (dd, J = 9 et 2,5 Hz : 1H) ; de 7,55 à 7,65 (mt : 1H) ; 7,73 (d, J = 2,5 Hz : 1H) ; 7,92 (d, J = 4,5 Hz : 1H) ; 8,02 (d, J = 9 Hz : 1H) ; 8,89 (d, J = 4,5 Hz : 1H).

## Revendications

1. Un dérivé de quinolyl propyl pipéridine, **caractérisé en ce qu'**il répond à la formule générale 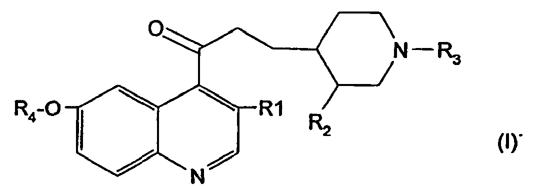 dans laquelle :
R₁ représente un atome d'hydrogène ou de fluor,
R₂ représente un radical carboxy, carboxyméthyle ou hydroxyméthyle,
R₃ représente un radical alcoyle (1 à 6 atomes de carbone) substitué par un radical phénylthio pouvant lui même porter 1 à 4 substituants choisis dans le groupe constitué par halogène, hydroxy, alcoyle, alcoyloxy, trifluorométhyle, trifluorométhoxy, carboxy, alcoyloxycarbonyle, cyano et amino, par un radical cycloalcoylthio dont la partie cyclique contient 3 à 7 chaînons pouvant lui-même porter 1 ou plusieurs substituants choisis parmi halogène et trifluorométhyle, ou par un radical hétéroarylthio de 5 à 6 chaînons comprenant 1 à 4 hétéroatomes choisis parmi l'azote, l'oxygène et le soufre, pouvant lui-même porter un ou plusieurs substituants choisis dans le groupe constitué par halogène, hydroxy, alcoyle, alcoyloxy, trifluorométhyle, trifluorométhoxy, carboxy, alcoyloxycarbonyle, cyano et amino ou R₃ représente un radical propargyle substitué par un radical phényle pouvant lui même porter 1 à 4 substituants choisis dans le groupe constitué par halogène, hydroxy, alcoyle, alcoyloxy, trifluorométhyle, trifluorométhoxy, carboxy, alcoyloxycarbonyle, cyano et amino, ou substitué par un radical cycloalcoyle contenant 3 à 7 chaînons, pouvant lui-même porter 1 ou plusieurs substituants choisis parmi halogène et trifluorométhyle, ou substitué par un radical hétéroaryle de 5 à 6 chaînons comprenant 1 à 4 hétéroatomes choisis parmi l'azote, l'oxygène et le soufre et pouvant lui-même porter un ou plusieurs substituants choisis dans le groupe constitué par halogène, hydroxy, alcoyle, alcoyloxy, trifluorométhyle, trifluorométhoxy, carboxy, alcoyloxycarbonyle, cyano et amino, et
R₄ représente un radical alcoyle contenant 1 à 6 atomes de carbone, alcényl-CH₂- ou alcynyl-CH₂- dont les parties alcényle ou alcynyle contiennent 2 à 6 atomes de carbone, cycloalcoyle ou cycloalcoyl alcoyle dont la partie cycloalcoyle contient 3 à 8 atomes de carbone,
sous ses différentes forme isomères, énantiomères et diastéréoisomères, séparées ou en mélanges, ainsi que ses sels.

2. Un dérivé de formule (I) telle que définie à la revendication 1, dans laquelle R₄ représente un radical alcoyle contenant de 1 à 6 atomes de carbone.

3. Un dérivé de formule (I) telle que définie à la revendication 1 ou 2, dans laquelle R₂ représente un radical carboxy.

4. Un dérivé de formule (I) telle que définie à l'une quelconque des revendications 1 à 3, dans laquelle R₃ représente un radical alcoyle renfermant de 1 à 6 atomes de carbone, -substitué par un radical phénylthio, cycloalcoylthio ou hétéroarylthio éventuellement substitué, tel que défini à la revendication 1.

5. Un dérivé de formule (I) telle que définie à l'une quelconque des revendications 1 à 4, dans laquelle R₃ représente un radical éthyle substitué par un radical thiénylthio, phénylthio substitué par halogène, cyclohexylthio ou cyclopentylthio.

6. L'un quelconque des dérivés de formule (I) telle que définie à la revendication 1, dont les noms suivent :
Acide 1-(2-cyclohexylsulfanyl-éthyl)-4-[3-(3-fluoro-6-méthoxy-quinolin-4-yl)-3-oxo-propyl]-pipéridine-3-carboxylique ;
Acide 4-[3-(3-fluoro-6-méthoxy-quinolin-4-yl)-3-oxo-propyl]-1-[3-(2,3,5-trifluoro-phényl)-prop-2-ynyl]-pipéridine-3-carboxylique ;
Acide 4-[3-oxo-3-(3-fluoro-6-méthoxyquinolin-4-yl)-propyl]-1-[2-(2,5-difluoro-phénylsulfanyl)-éthyl]-pipéridine-3-carboxylique ;
Acide 4-[3-oxo-3-(3-fluoro-6-méthoxyquinolin-4-yl)-propyl]-1-[2-(2,5-difluoro-phénylsulfanyl)-éthyl]-pipéridine-3-acétique ;
Acide 4-[3-oxo-3-(6-méthoxyquinolin-4-yl)-propyl]- 1-[2-(2-thiophènesulfanyl)-éthyl]-pipéridine-3-carboxylique ;
Acide 4-[3-oxo-3-(6-méthoxyquinolin-4-yl)propyl]-1-[3-(2,3,5-trifluorophényl)prop-2-ynyl]pipéridine-3-carboxylique ;
sous leurs différentes formes isomères, séparées ou en mélanges, et leur sels.

7. Un procécé de préparation de dérivé de quinolyl propyl pipéridine selon la revendication 1, **caractérisé en ce que** l'on condense la chaîne R₃ définie dans la revendication 1, sur le dérivé de quinolyl propyl pipéridine de formule générale (II) 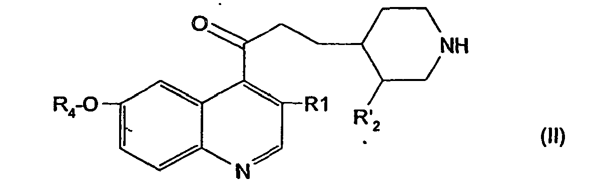 dans laquelle la fonction cétone est, le cas échéant, intermédiairement protégée, R₁ et R₄ sont définis comme dans la revendication 1 et R'₂ représente un radical carboxy ou carboxyméthyle protégé, pour obtenir un dérivé de quinolyl propyl pipéridine de formule générale (III) : 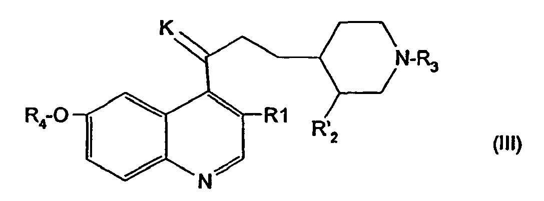 pour lequel R₁, R'₂, R₃ et R₄ sont définis comme ci-dessus et K représente un atome d'oxygène ou un groupement protecteur de cétone, puis transforme R'₂ en un radical carboxy ou carboxyméthyl, et/ou, le cas échéant, réduit le radical carboxy ainsi obtenu ou le radical carboxy protégé que peut représenter R'₂ en un radical hydroxyméthyle et éventuellement transformation de celui-ci en un radical carboxyméthyle selon les méthodes habituelles,
puis, le cas échéant, sépare les isomères, élimine le radical protecteur d'acide, élimine le radical protecteur de la cétone, et/ou transforme le produit obtenu en un sel.

8. Un procédé de préparation de dérivé de quinolyl propyl pipéridine selon la revendication 1, **caractérisé en ce que** l'on condense la chaîne R₃ définie dans la revendication 1, sur le dérivé de quinolyl propyl pipéridine de formule générale (II') : 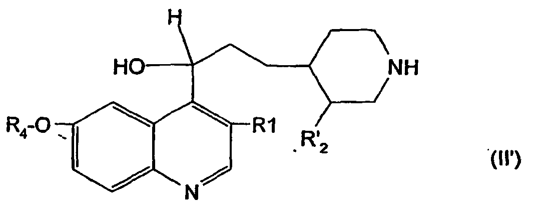 dans laquelle R₁ et R₄ sont définis comme dans la revendication 1 et R'₂ représente un radical carboxy ou carboxyméthyle protégé, pour obtenir un dérivé de quinolyl propyl pipéridine de formule générale (III'): 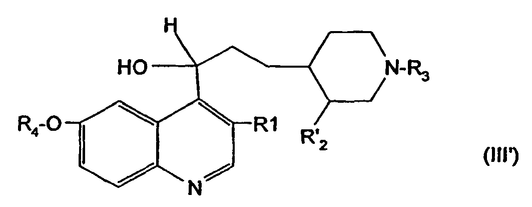 dans laquelle R₁, R'₃ et R₄ sont définis comme dans la revendication 1 et R₃ est défini comme précédemment, puis oxyde en cétone la fonction alcool en position alpha de la quinoléine pour obtenir un dérivé de formule (III) telle que définie à la revendication 7, dans laquelle K représente un atome d'oxygène, et poursuit la synthèse comme décrit à la revendication 7.

9. Procédé selon la revendication 7, **caractérisé en ce que** l'on prépare le dérivé de quinolyl propyl pipéridine de formule (II) pour lequel R₁ est un atome de fluor par un procédé selon lequel l'on condense le dérivé lithié en position 4 de la quinoléine (VI) : 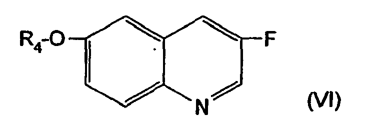 sur un dérivé de pipéridine de formule générale (VII) : 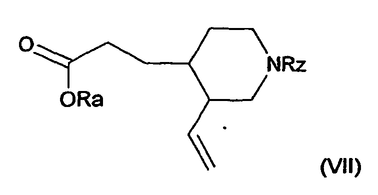 dans lequel Rz est un groupement protecteur d'amine et Ra est un groupement alkyle, pour obtenir un dérivé de quinolyl propyl pipéridine de formule générale (V) : 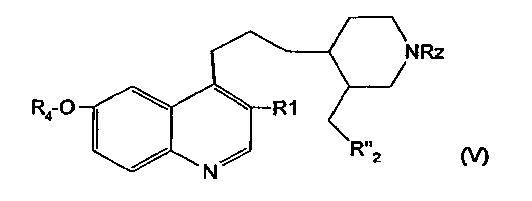 dans lequel Rz est défini comme précédemment, que l'on soumet à une réaction d'oxydation pour obtenir le dérivé carboxy correspondant, le cas échéant, protège le carboxy et la cétone, le cas échéant, réduit le carboxy libre ou protégé en hydroxyméthyle puis transforme celui-ci en carboxyméthyle, protège ce dernier et libère la cétone et l'amine.

10. A titre de médicaments, les dérivés de quinolyl propylpipéridine tels que définis à la revendication 1, ainsi que leurs sels pharmaceutiquement acceptables.

11. A titre de médicaments, les dérivés de quinolyl propylpipéridine tels que définis à l'une quelconque des revendications 2 à 6, ainsi que leurs sels pharmaceutiquement acceptables.

12. Compositions pharmaceutiques contenant, à titre de principe actif, au moins un médicament selon l'une des revendications 10 et 11.

13. Un dérivé de quinolyl pipéridine, **caractérisé en ce qu'**il répond à la formule générale (II) : 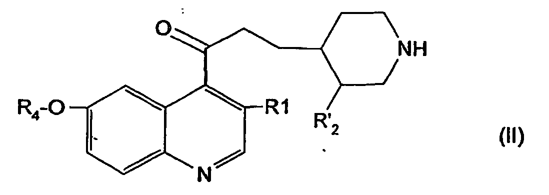 dans laquelle R4 est défini comme à la revendication 1, R₁ représente un atome de fluor et R'₂ est tel que défini à la revendication 7, ou à la formule correspondante dans laquelle la cétone est protégée.

14. Un dérivé de quinolyl propyl pipéridine, **caractérisé en ce qu'**il répond à la formule générale (A) : 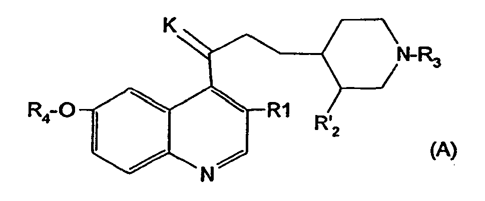 dans laquelle R1, R3 et R4 sont tels que définis à la revendication 1, R'2 est tel que défini à la revendication 7 et K représente un groupement protecteur de cétone.

15. Un dérivé de quinolyl propyl pipéridine, **caractérisé en ce qu'**il répond à la formule générale (B) : 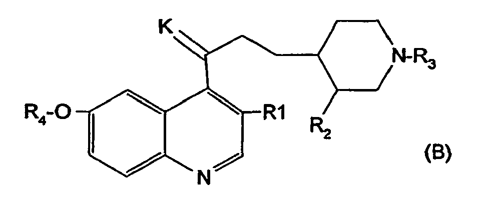 dans laquelle R1, R2, R3 et R4 sont tels que définis à la revendication 1 et K représente un groupement protecteur de cétone.

16. Un dérivé de quinolyl propyl pipéridine, **caractérisé en ce qu'**il répond à la formule générale (C) : 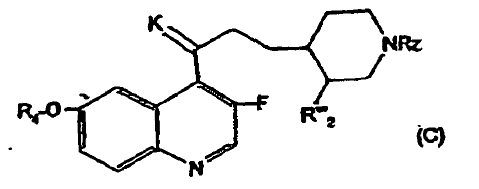 dans laquelle R₄ est tels que défini à la revendication 1, Rz représente un groupement protecteur d'amine, K représente un atome d'oxygène ou un groupement protecteur de cétone et R'''2 représente un radical carboxy ou carboxyméthyle libre ou protégé ou un radical hydroxyméthyle.

17. Un dérivé de quinolyl propyl pipéridine, **caractérisé en ce qu'**il répond à la formule générale (VIII) : 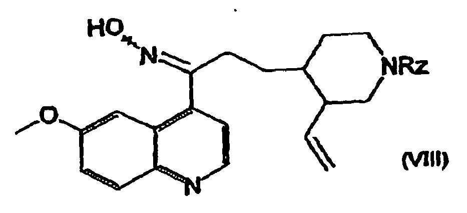 dans laquelle Rz est un groupement protecteur d'amine.

## Claims

1. A quinolylpropylpiperidine derivative, **characterized in that** it corresponds to the general formula 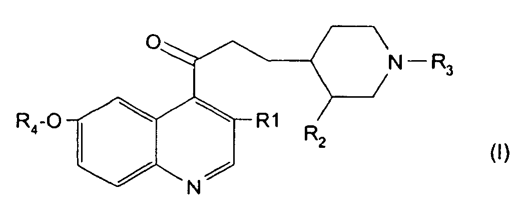 in which:
R₁ represents a hydrogen or a fluorine atom,
R₂ represents a carboxyl, carboxymethyl or hydroxymethyl radical,
R₃ represents an alkyl (1 to 6 carbon atoms) radical substituted with a phenylthio radical which may itself carry 1 to 4 substituents chosen from the group consisting of halogen, hydroxyl, alkyl, alkyloxy, trifluoromethyl, trifluoromethoxy, carboxyl, alkyloxycarbonyl, cyano and amino, with a cycloalkylthio radical in which the cyclic portion contains 3 to 7 members, which may itself carry 1 or more substituents chosen from halogen and trifluoromethyl, or with a 5- to 6-membered heteroarylthio radical comprising 1 to 4 heteroatoms chosen from nitrogen, oxygen and sulfur which may itself, carry one or more substituents chosen from the group consisting of halogen, hydroxyl, alkyl, alkyloxy, trifluoromethyl, trifluoromethoxy, carboxyl, alkyloxycarbonyl, cyano and amino or R₃ represents a propargyl radical substituted with a phenyl radical which may itself carry 1 to 4 substituents chosen from the group consisting of halogen, hydroxyl, alkyl, alkyloxy, trifluoromethyl, trifluoromethoxy, carboxyl, alkyloxycarbonyl, cyano and amino, or substituted with a 3- to 7-membered cycloalkyl radical which may itsef carry 1 or more substituents chosen from halogen and trifluoromethyl, or substituted with a 5- to 6-membered heteroaryl radical comprising 1 to 4 heteroatoms chosen from nitrogen, oxygen and sulfur and which may itself carry one or more substituents chosen from the group consisting of halogen, hydroxyl, alkyl, alkyloxy, trifluoromethyl, trifluoromethoxy, carboxyl, alkyloxycarbonyl, cyano and amino, and R₄ represents an alkyl radical containing 1 to 6 carbon atoms, an alkenyl-CH₂- or alkynyl-CH₂- radical in which the alkenyl or alkynyl portions contain 2 to 6 carbon atoms, or a cycloalkyl or cycloalkylalkyl radical in which the cycloalkyl portion contains 3 to 8 carbon atoms,
in its various isomeric, enantiomeric and diastereoisomeric forms, separate or as mixtures, as well as its salts.

2. A derivative of formula (I) as defined in claim 1, in which R⁴ represents an alkyl radical containing from 1 to 6 carbon atoms.

3. A derivative of formula (I) as defined in claim 1 or 2, in which R₂ represents a carboxyl radical.

4. A derivative of formula (I) as defined in any one of claims 1 to 3, in which R₃ represents an alkyl radical containing from 1 to 6 carbon atoms, substituted with an optionally substituted phenylthio, cycloalkylthio or heteroarylthio radical, as defined in claim 1.

5. A derivative of formula (I) as defined in any one of claims 1 to 4, in which R₃ represents an ethyl radical substituted with a thienylthio radical or a phenylthio radical substituted with halogen, cyclohexylthio or cyclopentylthio.

6. Any one of the derivatives of formula (I) as defined in claim 1, including the following names:
1-(2-cyclohexylsulfanyl-ethyl)-4-[3-(3-fluoro-6-methoxy-quinolin-4-yl)-3-oxo-propyl]-piperidine-3-carboxylic acid,
4-[3-(3-fluoro-6-methoxy-quinolin-4-yl)-3-oxo-propyl]-1-[3-(2,3,5-trifluoro-phenyl)prop-2-ynyl]-piperidine-3-carboxylic acid,
4-[3-oxo-3-(3-fluoro-6-methoxyquinolin-4-yl)propyl]-1-[2-(2,5-difluoro-phenylsulfanyl)-ethyl]piperidine-3-carboxylic acid,
4-[3-oxo-3-(3-fluoro-6-methoxyquinolin-4-yl)propyl]-1-[2-(2,5-difluoro-phenylsulfanyl)-ethyl]piperidine-3-acetic acid,
4-[3-oxo-3-(6-methoxyquinolin-4-yl)propyl]-1-[2-(2-thienylsulfanyl)ethyl]-piperidine-3-carboxylic acid,
4-[3-oxo-3-(6-methoxyquinolin-4-yl)propyl]-1-[3-(2,3,5-trifluorophenyl)prop-2-ynyl]piperidine-3-carboxylic acid,
in their various isomeric forms, separate or as mixtures, as well as their salts.

7. A process for preparing a quinolylpropylpiperidine derivative as claimed in claim 1, **characterized in that** the R₃ chain defined in claim 1 is condensed with the quinolylpropylpiperidine derivative of general formula (II) 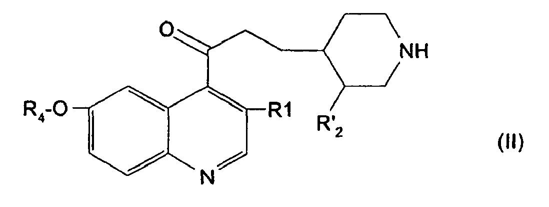 in which the ketone functional group is, where appropriate, intermediately protected, R₁ and R₄ are as defined in claim 1 and R'₂ represents a protected carboxyl or carboxymethyl radical, to obtain a quinolylpropylpiperidine derivative of general formula (III): 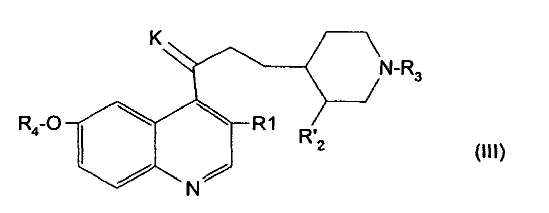 for which R₁, R'₂, R₃ and R₄ are as defined above and K represents an oxygen atom or a ketone-protecting group, then R'₂ is converted to a carboxyl or carboxymethyl radical, and/or, where appropriate, the carboxyl radical thus obtained or the protected carboxyl radical which may be represented by R'₂ is reduced to a hydroxymethyl and, optionally, said hydroxymethyl radical is converted to a carboxymethyl radical according to the usual methods,
then, where appropriate, the isomers are separated, the acid-protecting radical is removed, the ketone-protecting radical is removed, and/or the product obtained is converted to a salt.

8. A process for preparing a quinolylpropylpiperidine derivative as claimed in claim 1, **characterized in that** the R₃ chain defined in claim 1 is condensed with the quinolylpropylpiperidine derivative of general formula (II'): 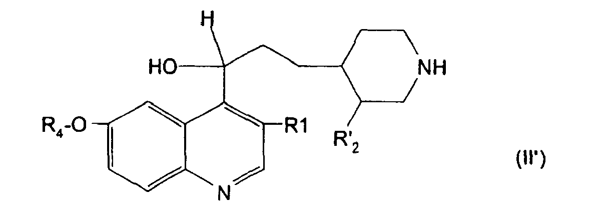 in which R₁ and R₄ are as defined in claim 1 and R'₂ represents a protected carboxyl or carboxymethyl radical, to obtain a quinolylpropylpiperidine derivative of general formula (III'): 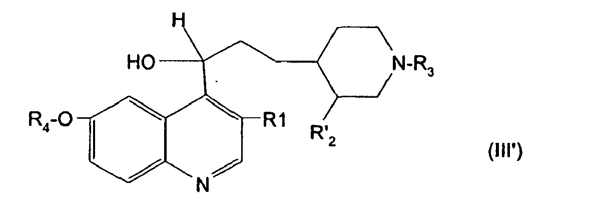 in which R₁, R'₃ and R₄ are as defined in claim 1 and R₃ is as defined above, then the alcohol functional group in the alpha position of the quinoline is oxidized to a ketone, to obtain a derivative of formula (III) as defined in claim 7, in which K represents an oxygen atom, and the synthesis is continued as described in claim 7.

9. The process as claimed in claim 7, **characterized in that** the quinolylpropylpiperidine derivative of formula (II) for which R₁ is a fluorine atom is prepared by a process according to which the derivative which is lithiated in the 4-position of the quinoline (VI) : 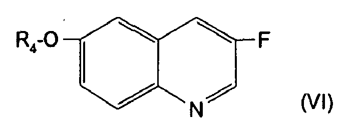 is condensed with a piperidine derivative of general formula (VII): 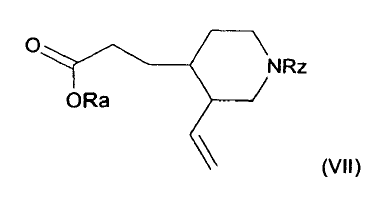 in which Rz is an amine-protecting group and Ra is an alkyl group, to obtain a quinolylpropyl-piperidine derivative of general formula (V): 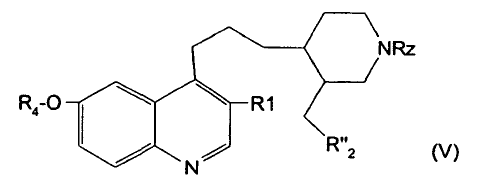 in which Rz is as defined above, which is subjected to an oxidation reaction to obtain the corresponding carboxyl derivative, where appropriate the carboxyl and the ketone are protected, where appropriate the free or protected carboxyl is reduced to hydroxymethyl, and then said hydroxymethyl is converted to carboxymethyl, the latter is protected and the ketone and the amine are freed.

10. As a medicament, the quinolylpropylpiperidine derivative as defined in claim 1, and also its pharmaceutically acceptable salts.

11. As a medicament, the quinolylpropylpipridine derivative as defined in any one of claims 2 to 6, and also its pharmaceutically acceptable salts.

12. A pharmaceutical composition containing, as active principle, at least one medicament as claimed in either of claims 10 and 11.

13. A quinolylpropylpiperidine derivative which corresponds to general formula (II): 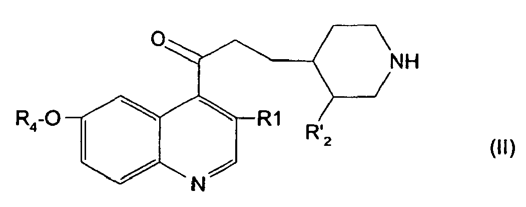 in which R₄ is as defined in claim 1, R₁ represents a fluorine atom and R'₂ is as defined in claim 7, or to the corresponding formula in which the ketone is protected.

14. A quinolylpropylpiperidine derivative, **characterized in that** it corresponds to general formula (A): 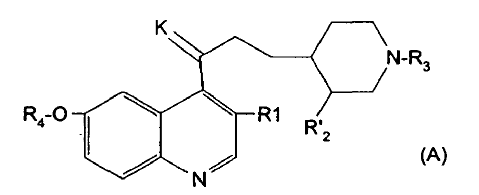 in which R1, R3 and R4 are as defined in claim 1, R'2 is as defined in claim 7 and K represents a ketone-protecting group.

15. A quinolylpropylpiperidine derivative, **characterized in that** it corresponds to general formula (B) : 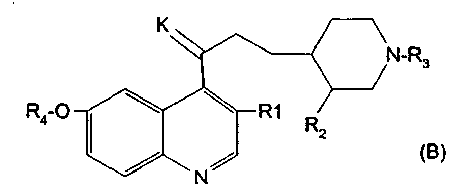 in which R1, R2, R3 and R4 are as defined in claim 1 and K represents a ketone-protecting group

16. A quinolylpropylpiperidine derivative, **characterized in that** it corresponds to the general formula (C): 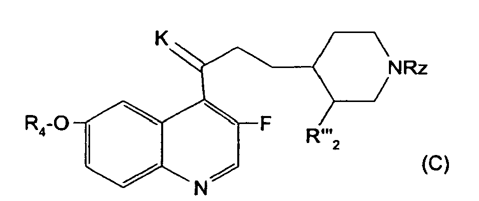 in which R₄ is as defined in claim 1, Rz represents an amine-protecting group, K represents an oxygen atom or a ketone-protecting group and R'''₂ represents a free or protected carboxyl or carboxymethyl radical or a hydroxymethyl radical.
in which Rz is an amine-protecting group and Ra is an alkyl group containing from 1 to 4 carbon atoms.

17. A quinolylpropylpiperidine derivative, **characterized in that** it corresponds to general formula (VIII): 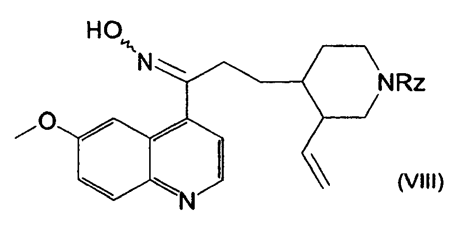 in which Rz is an amine-protecting group.

## Patentansprüche

1. Chinolylpropylpiperinderivat, **dadurch gekennzeichnet, dass** es der allgemeinen Formel 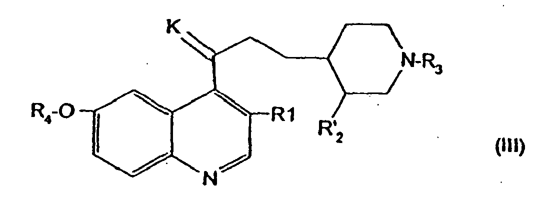 gehorcht, wobei
R₁ ein Wasserstoff- oder Fluoratom darstellt,
R₂ einen Carboxy-, Carboxymethyl- oder Hydroxymethylrest darstellt,
R₃ einen Alkylrest (1 bis 6 Kohlenstoffatome) darstellt, der substituiert ist mit einem Phenylthiorest, der selbst 1 bis 4 Substituenten tragen kann, die aus der Gruppe ausgewählt sind bestehend aus, Halogen, Hydroxy, Alkyl, Alkyloxy, Trifluormethyl, Trifluormethoxy, Carboxy, Alkyloxycarbonyl, Cyano und Amino, mit einem Cycloalkylthiorest, dessen cyclischer Teil 3 bis 7 Kettenglieder enthält, die selbst einen oder mehrere Substituenten tragen können, die aus Halogen und Trifluormethyl ausgewählt sind, oder mit einem Heteroarylthiorest mit 5 bis 6 Kettengliedern, die 1 bis 4 Heteroatome umfassen, die aus Stickstoff, Sauerstoff und Schwefel ausgewählt sind, die selbst einen oder mehrere Substituenten tragen können, die aus der Gruppe ausgewählt sind, bestehend aus Halogen, Hydroxy, Alkyl, Alkyloxy, Trifluormethyl, Trifluormethoxy, Carboxy, Alkyloxycarbonyl, Cyano und Amino, oder R₃ einen Propargylrest darstellt, der mit einem Phenylrest substituiert ist, der selbst 1 bis 4 Substituenten tragen kann, die aus der Gruppe ausgewählt sind, bestehend aus Halogen, Hydroxy, Alkyl, Alkyloxy, Trifluormethyl, Trifluormethoxy, Carboxy, Alkyloxycarbonyl, Cyano und Amino, oder mit einem Cycloalkylrest substituiert ist, der 3 bis 7 Kettenglieder enthält, die selbst 1 oder mehrere Substituenten tragen können, die aus Halogen und Trifluormethyl ausgewählt sind, oder mit einem Heteroarylrest von 5 bis 6 Kettengliedern substituiert ist, die 1 bis 4 Heteroatome umfassen, die aus Stickstoff, Sauerstoff und Schwefel ausgewählt sind, und die selbst einen oder mehrere Substituenten tragen können, die aus der Gruppe ausgewählt sind, bestehend aus Halogen, Hydroxy, Alkyl, Alkyloxy, Trifluormethyl, Trifluormethoxy, Carboxy, Alkyloxycarbonyl, Cyano und Amino und
R₄ einen Alkylrest, der 1 bis 6 Kohlenstoffatome enthält, Alkenyl-CH₂- oder Alkinyl-CH₂-, wovon die Alkenyl- oder Alkinylteile 2 bis 6 Kohlenstoffatome enthalten, Cycloalkyl oder Cycloalkylalkyl, wovon der Cycloalkylteil 3 bis 8 Kohlenstoffatome enthält, darstellt,
in seinen verschiedenen isomeren, enantiomeren und diastereoisomeren Formen, getrennt oder im Gemisch, sowie seine Salze.

2. Derivat der Formel (I) nach Anspruch 1, wobei R₄ einen Alkylrest darstellt, der 1 bis 6 Kohlenstoffatome enthält.

3. Derivat der Formel (I) nach Anspruch 1 oder 2, wobei R₂ einen Carboxyrest darstellt.

4. Derivat der Formel (I) nach einem der Ansprüche 1 bis 3, wobei R₃ einen Alkylrest darstellt, der 1 bis 6 Kohlenstoffatome einschließt, die mit einem Phenylthio-, Cycloalkylthio- oder Heteroarylthiorest, der gegebenenfalls wie in Anspruch 1 definiert substituiert ist, substituiert sind.

5. Derivat der Formel (I) nach einem der Ansprüche 1 bis 4, wobei R₃ einen Ethylrest darstellt, der mit einem Thienylthio-, Phenylthiorest substituiert ist, der mit Halogen, Cyclohexylthio oder Cyclopentylthio substituiert ist.

6. Eines der Derivate der Formel (I) nach Anspruch 1 mit den folgenden Namen:
1-(2-Cyclohexylsulfanyl-ethyl)-4-[3-(3-fluor-6-methoxy-chinolin-4-yl)-3-oxo-propyl]-piperidin-3-carbonsäure;
4-[3-(3-Fluor-6-methoxy-chinolin-4-yl)-3-oxo-propyl]-1-[3-(2,3,5-trifluorphenyl)-prop-2-inyl]-piperidin-3-carbonsäure;
4-[3-Oxo-3-(3-fluor-6-methoxychinolin-4-yl)-propyl]-1-(2-(2,5-difluor-phenylsulfanyl)-ethyl]-piperidin-3-carbonsäure;
4-[3-Oxo-3-(3-fluor-6-methoxychinolin-4-yl)-propyl]-1-[2-(2,5-difluor-phenylsulfanyl)-ethyl]-piperidin-3-essigsäure;
4-(3-Oxo-3-(6-methoxychinolin-4-yl)-propyl]-1-[2-(2-thiophensulfanyl)-ethyl]-piperidin-3-carbonsäure;
4-[3-Oxo-3-(6-methoxychinolin-4-yl)propyl]-1-[3-(2,3,5-trifluorphenyl)-prop-2-inyl]piperidin-3-carbonsäure;
in ihren verschiedenen isomeren Formen, getrennt oder im Gemisch, und ihre Salze.

7. Verfahren zur Herstellung eines Chinolylpropylpiperinderivats nach Anspruch 1, **dadurch gekennzeichnet, dass** die R₃-Kette, die in Anspruch 1 definiert ist, mit dem Chinolylpropylpiperidinderivat der allgemeinen Formel (II) 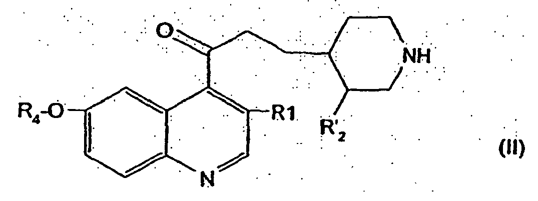 wobei die Ketonfunktion gegebenenfalls vorübergehend geschützt ist, R₁ und R₄ wie in Anspruch 1 definiert sind und R'₂ einen geschützten Carboxy- oder Carboxymethylrest darstellt, kondensiert wird, um ein Chinolylpropylpiperidinderivat der allgemeinen Formel (III) 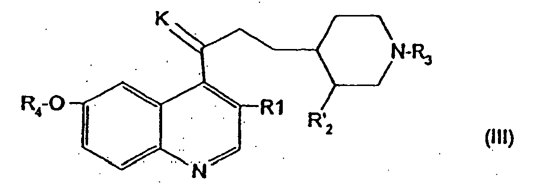 wobei R₁, R'₂, R₃ und R₄ wie vorstehend definiert sind und K ein Sauerstoffatom oder eine Keton-Schutzgruppe darstellt, zu erhalten, anschließend R'₂ in einen Carboxy- oder Carboxymethylrest umgewandelt und/oder der so erhaltene Carboxyrest oder der geschützte Carboxyrest, der R'₂ darstellen kann, gegebenenfalls zu einem Hydroxymethylrest reduziert wird und dieser gegebenenfalls nach den üblichen Verfahren in einen Carboxymethylrest übergeführt wird,
die Isomere anschließend gegebenenfalls getrennt werden, die SäureSchutzgruppe entfernt wird, die Keton-Schutzgruppe entfernt wird und/oder das erhaltenen Produkt in ein Salz übergeführt wird.

8. Verfahren zur Herstellung eines Chinolylpropylpipendinderivats nach Anspruch 1, **dadurch gekennzeichnet, dass** die in Anspruch 1 definierte R₃-Kette mit dem Chinolylpropylpiperidinderivat der allgemeinen Formel (II') 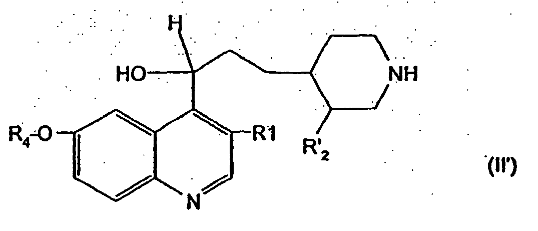 wobei R₁ und R₄ wie in Anspruch 1 definiert sind und R'₂ einen geschützten Carboxy- oder Carboxymethylrest darstellt, kondensiert wird, um einen Chinolylpropylpiperidinrest der allgemeinen Formel (III'): 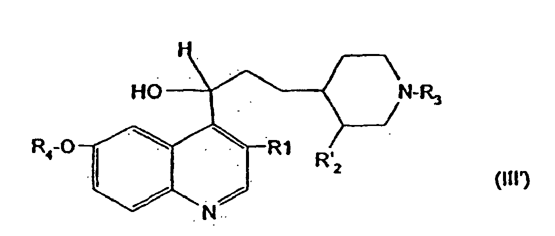 wobei R₁, R'₃ und R₄ wie in Anspruch 1 definiert sind und R₃ wie zuvor definiert ist, zu erhalten, anschließend die Alkoholfunktion in α-Position des Chinolins zum Keton oxidiert wird, um ein Derivat der Formel (III), wie in Anspruch 7 definiert, zu erhalten, wobei K ein Sauerstoffatom darstellt, und die Synthese wie in Anspruch 7 beschrieben fortgeführt wird.

9. Verfahren nach Anspruch 7, **dadurch gekennzeichnet, dass** das Chinolylpropylpiperidinderivat der Formel (II), wobei R₁ ein Fluoratom ist, durch ein Verfahren hergestellt wird, wobei das in Position 4 des Chinolins (VI): 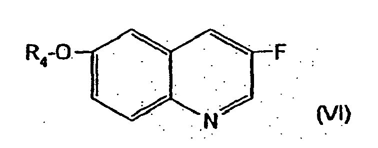 lithüerte Derivat mit einem Piperidinderivat der allgemeinen Formel (VII): 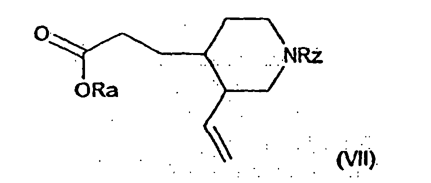 wobei Rz eine Aminschutzgruppe ist und Ra eine Alkylgruppe ist, kondensiert wird, um ein Chinolylpropylpiperidin-Derivat der allgemeinen Formel (V) 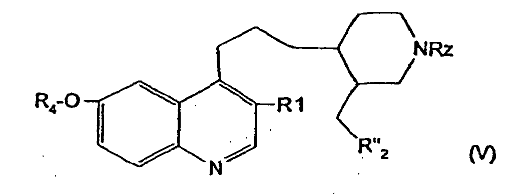 wobei Rz wie zuvor definiert ist, zu erhalten, mit dem eine Oxidationsreaktion durchgeführt wird, um das entsprechende Carboxyderivat zu erhalten, das Carboxy und das Keton gegebenenfalls geschützt werden, das freie oder geschützte Carboxy gegebenenfalls zu Hydroxymethyl reduziert wird, dieses anschließend in Carboxymethyl übergeführt wird, das Letztere geschützt wird und das Keton und das Amin freigesetzt werden.

10. Die Chinolylpropylpiperidinderivate nach Anspruch 1 sowie ihre pharmazeutisch verträglichen Salze als Medikament.

11. Die Chinolylpropylpiperidinderivate nach einem der Ansprüche 2 bis 6 sowie ihre pharmazeutisch verträglichen Salze als Medikament.

12. Pharmazeutische Zubereitungen, die als Wirkstoff mindestens ein Medikament nach einem der Ansprüche 10 und 11 enthalten.

13. Chinolylpropylpiperidinderivat, **dadurch gekennzeichnet dass** es der allgemeinen Formel (II) 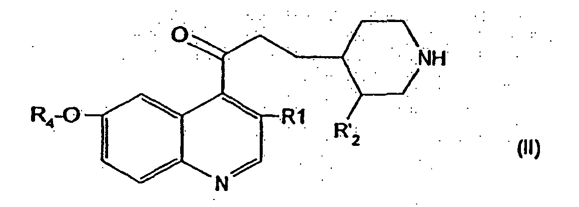 wobei R₄ wie in Anspruch 1 definiert ist, R₁ ein Fluoratom darstellt und R'₂ wie in Anspruch 7 definiert ist, oder der entsprechenden Formel, wobei das Keton geschützt ist, gehorcht.

14. Chinolylpropylpiperidinderivat, **dadurch gekennzeichnet, dass** es der allgemeinen Formel (A) 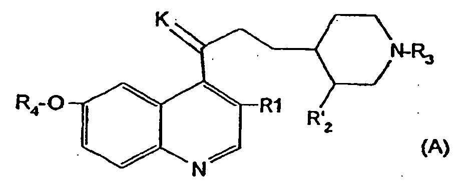 wobei R₁, R₃ und R₄ wie in Anspruch 1 definiert sind, R'₂ wie in Anspruch 7 definiert ist und K eine Keton-Schutzgruppe darstellt, gehorcht.

15. Chinolylpropylpiperidinderivat, **dadurch gekennzeichnet, dass** es der allgemeinen Formel (B): 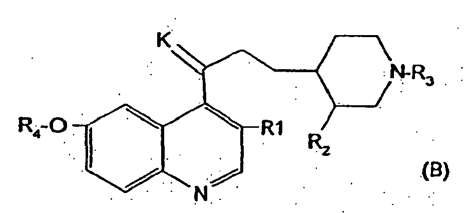 wobei R₁, R₂, R₃ und R₄ wie in Anspruch 1 definiert sind und K eine Keton-Schutzgruppe darstellt, gehorcht.

16. Chinolylpropylpiperidinderivat, **dadurch gekennzeichnet, dass** es der allgemeinen Formel (C): 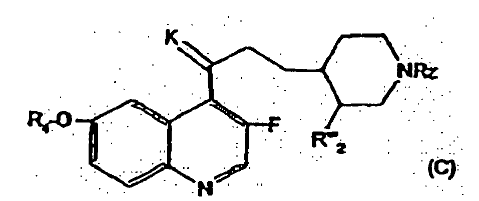 wobei R₄ wie in Anspruch 1 definiert ist, Rz eine Aminschutzgruppe darstellt, K ein Sauerstoffatom oder eine Keton-Schutzgruppe darstellt und R‴₂ einen freien oder geschützten Carboxy- oder Carboxymethylrest oder einen Hydroxymethylrest darstellt, gehorcht.

17. Chinolylpropylpiperidinderivat, **dadurch gekennzeichnet, dass** es der allgemeinen Formel (VIII): 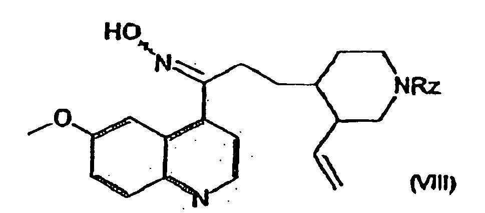 wobei Rz eine Amin-Schutzgruppe ist, gehorcht.
